# EUROPEAN PATENT APPLICATION

(11) **EP 4 650 338 A1**
(43) Date of publication of application: **19.11.2025**
(21) Application number: 23915829.8
(22) Date of filing: 28.12.2023
(51) Int. Cl.: C07C 4/06, C10G 21/02, B01J 8/24

(54) **FLUIDIZED CATALYTIC CONVERSION SYSTEM AND USE THEREOF**

(30) Priority: 09.01.2023 CN 202310029764; 09.01.2023 CN 202310037752; 09.01.2023 CN 202310029168; 09.01.2023 CN 202310037661
(71) Applicant: China Petroleum & Chemical Corporation, Beijing 100728 (CN); Sinopec Research Institute of Petroleum Processing Co., Ltd., Beijing 100083 (CN)
(72) Inventor: MA, Wenming, Beijing 100083 (CN); YUAN, Qimin, Beijing 100083 (CN); ZHU, Genquan, Beijing 100083 (CN)
(74) Representative: karo IP
(86) International application number: PCT/CN2023/142632
(87) International publication number: WO 2024/149076

(57) **Abstract**

A fluidized catalytic conversion system is disclosed, which comprises a reaction unit, a catalyst separation unit, and a catalyst regeneration unit. The reaction unit comprises a first fluidized bed reactor and a second fluidized bed reactor connected in series, or comprises a composite fluidized bed reactor having a first reaction zone and a second reaction zone connected in series. The catalyst separation unit comprises a disengager and a catalyst separator arranged inside or outside the disengager. The catalyst regeneration unit comprises a first catalyst regenerator and a second catalyst regenerator, or comprises a composite catalyst regenerator provided with a first regeneration zone and a second regeneration zone. The fluidized catalytic conversion system can allow the reaction raw materials to undergo different reactions in different reactors or reaction zones under more suitable catalysts and reaction conditions, thereby improving the yield of the target products.

## Description

### Technical Field

The present application relates to the field of petrochemical engineering, and in particular to a fluidized catalytic conversion system and its application.

### Background Art

In the petrochemical production process, different reaction environments can be constructed by using multiple reactors or multiple reaction zones. At the same time, the catalysts can be separated to achieve the use of suitable catalysts for different reactions, or different regeneration zones can be used to regenerate the catalysts, thereby increasing the yield of the target product.

CN110317628A discloses a catalyst zoned integrated catalytic cracking method and device. The device comprises a main reactor, a secondary reactor, a regenerator, and an A catalyst storage tank and a B catalyst storage tank, wherein an A catalyst primary cyclone separator and a B catalyst primary cyclone separator are arranged at the upper portion in the them. The regenerator is connected to the A catalyst primary cyclone separator, and the A catalyst primary cyclone separator is connected to the B catalyst primary cyclone separator. The B catalyst primary cyclone separator is connected to the regenerator. The bottom of the A catalyst storage tank is connected to the main reactor, and the bottom of the B catalyst storage tank is connected to the secondary reactor.

CN104560149A discloses a catalytic conversion method for producing butylene. The method has four reactors in total. In addition to adopting a reactor configuration of a double riser and a fluidized bed, a fluidized bed reactor is also arranged outside the disengager, for cracking the middle gasoline cut, and the reaction product enters the riser reactor to continue the cracking reaction. The catalyst after the reaction is charred and regenerated and then returned to the reactor for recycling. The method uses a mixture containing Y zeolite and β zeolite as a catalyst, and can obtain a higher yield of propylene and butylene.

CN107828443A discloses a fluidized catalytic cracking method and apparatus for maximizing light olefin yield and other applications, wherein hydrocarbons, a first catalyst and a second catalyst are fed into a reactor, wherein the first catalyst has a smaller average particle size and is less dense than the second catalyst. A first portion of the second catalyst is recovered as a bottom product of the reactor. Cracked hydrocarbon effluent, a second portion of the second catalyst and the first catalyst are recovered as a top product of the reactor. The top product is separated in a separation system to obtain a first stream containing the first catalyst and the hydrocarbon effluent and a second stream containing the second catalyst. The second catalyst separated in the second stream is returned to the reactor.

CN1031834A discloses a catalytic conversion method for producing light olefins. The method uses petroleum fractions, residual oil or feedstock oil with different boiling ranges as raw materials. A mixture containing Y zeolite and five-membered ring high-silicon zeolite is used as catalyst. A fluidized bed or a moving bed is used as a reactor. The reaction conditions are: temperature 500-650°C, pressure 0.15-0.30MPa, weight hourly space velocity 0.2-20 h⁻¹, catalyst-oil ratio 2-12, and the catalyst after the reaction is charred and regenerated and then returned to the reactor for recycling.

However, when the existing catalytic cracking conversion system and process are used to catalytically crack heavy feedstock oil to produce ethylene and propylene, the yield of ethylene and propylene obtained still needs to be further improved.

### Summary of the invention

The purpose of the present application is to provide a fluidized catalytic conversion system and its application, wherein the fluidized catalytic conversion system adopts multiple fluidized bed reactors or reaction zones connected in series, so that the reaction raw materials can undergo different reactions in different reactors or reaction zones under more suitable catalysts and reaction conditions, thereby improving the yield of the target products.

In order to achieve the above objectives, on the one hand, the present application provides a fluidized catalytic conversion system, comprising:
A reaction unit, in which the reaction raw materials are contacted with the catalyst to react to obtain an oil catalyst mixture. The reaction unit comprising a first fluidized bed reactor and a second fluidized bed reactor connected in series, or comprising a composite fluidized bed reactor having a first reaction zone and a second reaction zone connected in series. The first fluidized bed reactor or the first reaction zone of the composite fluidized bed reactor having a first feedstock oil inlet, a first catalyst inlet and a first oil catalyst mixture outlet. The second fluidized bed reactor or the second reaction zone of the composite fluidized bed reactor having an oil catalyst mixture inlet, a second catalyst inlet, a second oil catalyst mixture outlet and an optional second feedstock oil inlet, wherein the first oil catalyst mixture outlet of the first fluidized bed reactor or the first reaction zone of the composite fluidized bed reactor is connected to the oil catalyst mixture inlet of the second fluidized bed reactor or the second reaction zone of the composite fluidized bed reactor;
A catalyst separation unit, in which the oil catalyst mixture from the reaction unit is separated to obtain an oil and gas product and a spent spent catalyst. The catalyst separation unit comprising a disengager and a catalyst separator arranged inside or outside the disengager. The disengager comprising a gas-solid separator, at least one settling zone and at least one stripping section connected to the bottom of the settling zone, and having an oil catalyst mixture inlet, an oil and gas product outlet and at least one catalyst outlet arranged at the bottom of the stripping section. The catalyst separator has a material inlet, a first material outlet and a second material outlet, wherein the oil catalyst mixture inlet of the disengager is connected to the second oil catalyst mixture outlet of the second fluidized bed reactor or the second reaction zone of the composite fluidized bed reactor; and
A catalyst regeneration unit, in which the spent spent catalyst from the catalyst separation unit is regenerated and recycled back to the reaction unit. The catalyst regeneration unit includes a first catalyst regenerator and a second catalyst regenerator, or includes a composite catalyst regenerator provided with a first regeneration zone and a second regeneration zone. The first catalyst regenerator or the first regeneration zone of the composite catalyst regenerator has a first spent spent catalyst inlet and a first regenerated catalyst outlet. The second catalyst regenerator or the second regeneration zone of the composite catalyst regenerator has a second spent spent catalyst inlet and a second regenerated catalyst outlet, wherein the first and second spent spent catalyst inlets are respectively connected to a catalyst outlet of the disengager, or are respectively connected to the first and second material outlets of the catalyst separator. The first regenerated catalyst outlet is connected to the first catalyst inlet of the first fluidized bed reactor or the first reaction zone of the composite fluidized bed reactor. The second regenerated catalyst outlet is connected to the second catalyst inlet of the second fluidized bed reactor or the second reaction zone of the composite fluidized bed reactor.

On the other hand, a use of the fluidized catalytic conversion system of the present application for catalytic conversion of hydrocarbon oil, especially for catalytic conversion of heavy feedstock oil to produce light olefins is provided.

In another aspect, a method for catalytic conversion of hydrocarbon oil, especially heavy feedstock oil, using the fluidized catalytic conversion system of the present application is provided, comprising the following steps:
1) contacting the hydrocarbon oil feedstock with a first catalyst in a first fluidized bed reactor or a first reaction zone of a composite fluidized bed reactor of a reaction unit of the fluidized catalytic conversion system, to perform a first catalytic conversion reaction to obtain a first oil catalyst mixture;
2) contacting the first oil catalyst mixture with a second catalyst in a second fluidized bed reactor or a second reaction zone of a composite fluidized bed reactor of a reaction unit of the fluidized catalytic conversion system, to perform a second catalytic conversion reaction to obtain a second oil catalyst mixture;
3) separating the second oil catalyst mixture in a catalyst separation unit of the fluidized catalytic conversion system to obtain an oil and gas product, a first spent spent catalyst, and a spent second spent catalyst;
4) regenerating the first spent spent catalyst in the first catalyst regenerator or the first regeneration zone of the composite catalyst regenerator of the catalyst regeneration unit of the fluidized catalytic conversion system, and returning the obtained first regenerated catalyst to step 1) as the first catalyst; and
5) regenerating the second spent catalystspent catalyst in the second catalyst regenerator or the second regeneration zone of the composite catalyst regenerator of the catalyst regeneration unit of the fluidized catalytic conversion system, and returning the obtained second regenerated catalyst to step 2) as the second catalyst.

The fluidized catalytic conversion system of the present application adopts multiple fluidized bed reactors/reaction zones in series, constructs different reaction environments therein, and regenerates separately by adopting multiple catalysts, combining multi-stage catalyst separation and multiple catalyst regenerators/regeneration zones, so as to realize different reactions in different reactors/reaction zones under more suitable catalysts and reaction conditions, thereby improving the yield of the target products. In particular, when used for catalytic cracking of heavy feedstock oil to produce light olefins, the fluidized catalytic conversion system of the present application can further improve the yield of ethylene and propylene.

The catalytic conversion method of the present application firstly contacts the hydrocarbon oil feedstock with a first catalyst to carry out a first catalytic conversion reaction (such as a primary cracking reaction or an alkane dehydrogenation reaction), and then contacts the reaction effluent with a second catalyst to carry out a first catalytic conversion reaction (such as a secondary cracking reaction or an olefin cracking reaction), thereby realizing the relay of the two-step catalytic conversion reaction of the hydrocarbon oil feedstock, so that the active species generated in the first step reaction can be fully retained in the feed of the second step reaction, thereby facilitating the second catalytic conversion reaction and inhibiting the occurrence of side reactions, thereby increasing the yield of target products, such as light olefins such as ethylene and propylene.

Other features and advantages of the present application will be described in detail in the subsequent detailed description.

### BRIEF DESCRIPTION OF THE DRAWINGS

The accompanying drawings are used to provide a further understanding of the present application and constitute a part of the specification. Together with the following specific embodiments, the accompanying drawings are used to explain the present application but do not constitute a limitation to the present application. In the accompanying drawings:
FIG. 1 is a schematic structural diagram of a preferred embodiment of a reaction unit of the system of the present application;
FIG. 2 is a schematic structural diagram of another preferred embodiment of the reaction unit of the system of the present application;
FIG. 3 is a schematic structural diagram of another preferred embodiment of the reaction unit of the system of the present application;
FIG. 4 is a schematic structural diagram of a preferred embodiment of a catalyst separator of the system of the present application;
FIG. 5 is a schematic structural diagram of another preferred embodiment of the catalyst separator of the system of the present application;
FIG. 6 is a schematic structural diagram of another preferred embodiment of the catalyst separator of the system of the present application;
FIG. 7 is a schematic structural diagram of another preferred embodiment of the catalyst separator of the system of the present application;
FIG. 8 is a schematic structural diagram of a catalytic conversion system of an embodiment of the present application.
FIG. 9 is a schematic structural diagram of an embodiment of a catalyst separation unit of the present application.
FIG. 10 is a schematic structural diagram of an embodiment of a regenerator in the present application.
FIG. 11 is a schematic structural diagram of a catalytic conversion system of an embodiment of the present application.
FIG. 12 is a schematic structural diagram of an embodiment of a catalyst separation unit of the present application.
FIG. 13 is a schematic structural diagram of an embodiment of a regenerator of the present application.
FIG. 14 is a schematic structural diagram of a catalytic conversion system of an embodiment of the present application.
FIG. 15 is a schematic structural diagram of a catalytic conversion system of an embodiment of the present application.

### Description of Reference Numerals

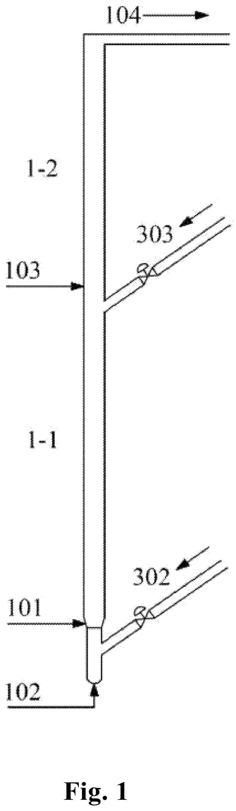

In Figure 1-15:
1-1 first reaction zone, 1-2 second reaction zone, 2 disengager, 2-1 (first) settling zone, 2-2 (first) stripping section , 2-21 stripping baffle, 2-3 second settling zone, 2-4 second stripping section, 2-41 stripping baffle, 2-50 catalyst separator, 2-51 first stage catalyst separator, 2-52 second stage catalyst separator, 2-6 settling zone baffle, 2-7 cyclone separator, 2-8 gas collecting chamber, 3-1 first regeneration zone, 3-2 second regeneration zone, 3-3 regenerator partition, 3-41 cyclone separator, 3-42 cyclone separator, 3-5 gas collecting chamber;
101 first feedstock oil, 102 pre-lifting gas, 103 second feedstock oil, 104 second oil catalyst mixture, 200 delivery pipe for spent catalyst, 201 stripping gas, 202 delivery pipe for first spent catalystspent catalyst, 203 stripping product, 204 second catalyst delivery pipe, 205 stripping gas, 206 delivery pipe for second spent catalystspent catalyst, 207 stripping product, 208 reaction oil and gas, 301A main air, 301B main air, 302 delivery pipe for first regeneration catalyst, 303 delivery pipe for second regeneration catalyst, 304A regenerated flue gas, 304B regenerated flue gas.

### DETAILED DESCRIPTION

The specific implementation of the present application is described in detail below. It should be understood that the specific implementation described here is only used to illustrate and explain the present application, and is not used to limit the present application.

The specific implementation of the present application is described in detail below. It should be understood that the specific implementation described here is only used to illustrate and explain the present application, and is not used to limit the present application.

Any specific numerical value disclosed herein (including the endpoint of the numerical range) is not limited to the exact value of the numerical value, but should be understood to also cover values close to the exact value, such as all possible values within the range of ±5% of the exact value. In addition, for the disclosed numerical range, the range between the endpoint values, the range between the endpoint values and the specific point values , and the range between specific point values can be arbitrarily combined to obtain one or more new numerical ranges, and these new numerical ranges should also be deemed to be specifically disclosed herein.

Unless otherwise specified, the terms used herein have the same meaning as commonly understood by those skilled in the art. If a term is defined herein and its definition is different from the commonly understood meaning in the art, the definition herein shall prevail.

In the present application, an "upflow bed" refers to a fluidized bed in which the reaction raw materials and the catalyst move from bottom to top; a "downflow bed" refers to a fluidized bed in which the reaction raw materials and the catalyst move from top to bottom.

In this application, except for the contents clearly stated, any matters or items not mentioned are directly applicable to those known in the art without any changes. Moreover, any embodiment described herein can be freely combined with one or more other embodiments described herein, and the technical solutions or technical ideas formed thereby are deemed to be part of the original disclosure or original record of this application, and should not be regarded as new contents not disclosed or anticipated herein, unless a person skilled in the art considers that the combination is obviously unreasonable.

All patent and non-patent literature, including but not limited to textbooks and journal articles, mentioned herein are incorporated herein by reference in their entirety.

As described above, in a first aspect, the present application provides a fluidized catalytic conversion system, comprising:
A reaction unit, in which the reaction raw materials are contacted with the catalyst to react to obtain an oil catalyst mixture. The reaction unit comprising a first fluidized bed reactor and a second fluidized bed reactor connected in series, or comprising a composite fluidized bed reactor having a first reaction zone and a second reaction zone connected in series. The first fluidized bed reactor or the first reaction zone of the composite fluidized bed reactor have a first feedstock oil inlet, a first catalyst inlet and a first oil catalyst mixture outlet. The second fluidized bed reactor or the second reaction zone of the composite fluidized bed reactor having an oil catalyst mixture inlet, a second catalyst inlet, a second oil catalyst mixture outlet and an optional second feedstock oil inlet, wherein the first oil catalyst mixture outlet of the first fluidized bed reactor or the first reaction zone of the composite fluidized bed reactor is connected to the oil catalyst mixture inlet of the second fluidized bed reactor or the second reaction zone of the composite fluidized bed reactor;
A catalyst separation unit, in which the oil catalyst mixture from the reaction unit is separated to obtain an oil and gas product and a spent catalystspent catalyst. The catalyst separation unit comprises a disengager and a catalyst separator arranged inside or outside the disengager. The disengager comprises a gas-solid separator, at least one settling zone and at least one stripping section connected to the bottom of the settling zone, and has an oil catalyst mixture inlet, an oil and gas product outlet and at least one catalyst outlet arranged at the bottom of the stripping section. The catalyst separator has a material inlet, a first material outlet and a second material outlet, wherein the oil catalyst mixture inlet of the disengager is connected to the second oil catalyst mixture outlet of the second fluidized bed reactor or the second reaction zone of the composite fluidized bed reactor; and
A catalyst regeneration unit, in which the spent catalyst from the catalyst separation unit is regenerated and recycled back to the reaction unit. The catalyst regeneration unit includes a first catalyst regenerator and a second catalyst regenerator, or includes a composite catalyst regenerator provided with a first regeneration zone and a second regeneration zone. The first catalyst regenerator or the first regeneration zone of the composite catalyst regenerator has a first spent catalyst inlet and a first regenerated catalyst outlet. The second catalyst regenerator or the second regeneration zone of the composite catalyst regenerator has a second spent catalyst inlet and a second regenerated catalyst outlet, wherein the first and second spent catalyst inlets are respectively connected to a catalyst outlet of the disengager, or are respectively connected to the first and second material outlets of the catalyst separator. The first regenerated catalyst outlet is connected to the first catalyst inlet of the first fluidized bed reactor or the first reaction zone of the composite fluidized bed reactor. The second regenerated catalyst outlet is connected to the second catalyst inlet of the second fluidized bed reactor or the second reaction zone of the composite fluidized bed reactor.

In certain preferred embodiments, in the catalyst separation unit, the catalyst separator is arranged outside the disengager. The catalyst outlet of the disengager is connected to the material inlet of the catalyst separator. The first material outlet of the catalyst separator is connected to the first spent catalyst inlet of the first catalyst regenerator or the first regeneration zone of the composite catalyst regenerator. The second material outlet is connected to the second spent catalyst inlet of the second catalyst regenerator or the second regeneration zone of the composite catalyst regenerator.

In other preferred embodiments, in the catalyst separation unit, the catalyst separator is arranged inside the disengager. The disengager has a first settling zone, a first stripping section connected to the bottom of the first settling zone, a first catalyst outlet arranged at the bottom of the first stripping section, a second settling zone, a second stripping section connected to the bottom of the second settling zone, and a second catalyst outlet arranged at the bottom of the second stripping section. The second oil catalyst mixture outlet of the second fluidized bed reactor or the second reaction zone of the composite fluidized bed reactor is connected to the material inlet of the catalyst separator via the oil catalyst mixture inlet of the disengager. The first material outlet and the second material outlet of the catalyst separator are respectively connected to the first settling zone and the second settling zone of the disengager. The first catalyst outlet of the disengager is connected to the first spent catalyst inlet in the first catalyst regenerator or the first regeneration zone of the composite catalyst regenerator. The second catalyst outlet of the disengager is connected to the second spent catalyst inlet in the second catalyst regenerator or the second regeneration zone of the composite catalyst regenerator.

In a preferred embodiment, the fluidized catalytic conversion system further includes a first catalyst injected into the first fluidized bed reactor or the first reaction zone of the composite fluidized bed reactor through the first catalyst inlet, and a second catalyst injected into the second fluidized bed reactor or the second reaction zone of the composite fluidized bed reactor through the second catalyst inlet, wherein the particle size and density of the first catalyst are respectively greater than the particle size and density of the second catalyst.

In a preferred embodiment, the first fluidized bed reactor and the second fluidized bed reactor, or the first reaction zone and the second reaction zone of the composite fluidized bed reactor, are independently of each other in the form of an upflow bed or a downflow bed, and more preferably, both are in the form of an upflow bed.

In a further preferred embodiment, the upward bed is the type of one or a combination of multiple selected from a bubbling bed, a turbulent bed, a fast bed and a dilute transport bed, preferably a type selected from a riser reactor, a fast bed reactor or a dense phase bed reactor.

In certain further preferred embodiments, the second fluidized bed reactor or the second reaction zone of the composite fluidized bed reactor is of an upflow bed type, and is partially or entirely disposed in at least one settling zone of the disengager.

In a particularly preferred embodiment, the first fluidized bed reactor and the second fluidized bed reactor, or the first reaction zone and the second reaction zone of the composite fluidized bed reactor, are independently of each other an upflow bed type selected from a riser reactor, a fast bed reactor or a dense phase bed reactor.

The first fluidized bed reactor or the first reaction zone of the composite fluidized bed reactor is provided with the first feedstock oil inlet, the first catalyst inlet and the fluidized medium inlet at the lower portion, and is provided with the first oil catalyst mixture outlet at the upper portion; and
The second fluidized bed reactor or the second reaction zone of the composite fluidized bed reactor is provided with the oil catalyst mixture inlet, the second catalyst inlet, and the optional second feedstock oil inlet at the lower portion, and is provided with the second oil catalyst mixture outlet at the upper portion.

As shown in FIG. 1, in certain particularly preferred embodiments, the first reaction zone 1-1 and the second reaction zone 1-2 of the composite fluidized bed reactor are both of the type of riser reactors with constant diameters.

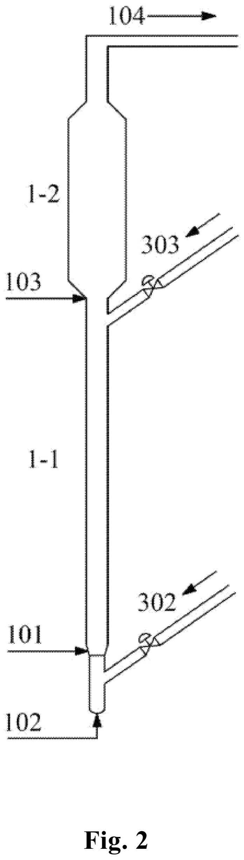

As shown in FIG. 2, in some other particularly preferred embodiments, the first reaction zone 1 - 1 of the composite fluidized bed reactor is of the type of a riser reactor with a constant diameter, and the second reaction zone 1 - 2 is of the type of a fast bed reactor.

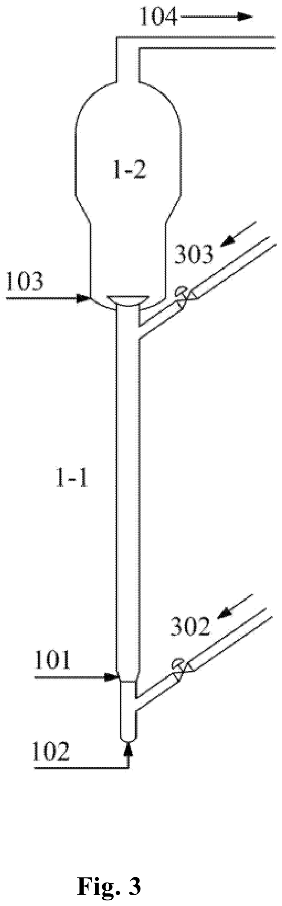

FIG. 3, in some other particularly preferred embodiments, the first reaction zone 1 - 1 of the composite fluidized bed reactor is of the type of a constant diameter riser reactor, and the second reaction zone 1 - 2 is of the type of a dense phase bed reactor.

In the embodiment shown in FIG. 1-3, the first regenerated catalyst from the first catalyst regenerator or the first regeneration zone of the composite catalyst regenerator is introduced into the bottom of the first reaction zone 1-1 through the first regenerated catalyst delivery pipe 302, and contacts and reacts with the injected first feedstock oil 101 after being lifted by the pre-lifting gas 102. The generated first oil catalyst mixture enters the bottom of the second reaction zone 1-2, and the second regenerated catalyst from the second catalyst regenerator or the second regeneration zone of the composite catalyst regenerator is introduced into the bottom of the second reaction zone 1-2 through the second regenerated catalyst delivery pipe 303, and contacts and reacts with the first oil catalyst mixture from the first reaction zone and the optionally injected second feedstock oil 103 to obtain the second oil catalyst mixture 104 .

In certain preferred embodiments, the catalyst separator is one or a combination of multiple selected from a cyclone-type fast separator, a tri-lobe fast separator, a ballistic fast separator, a U-tube separator, and a wall-cutting fast separator, preferably a cyclone-type fast separator.

In other preferred embodiments, the catalyst separator is configured in the form of multiple separators in parallel, or in the form of a multi-stage separator in series, or a combination thereof, wherein each separator or each stage of the multi-stage separator is independently a cyclone-type fast separator, a tri-lobe fast separator, a ballistic fast separator, a U-tube separator or a wall-cutting fast separator, preferably a cyclone-type fast separator.

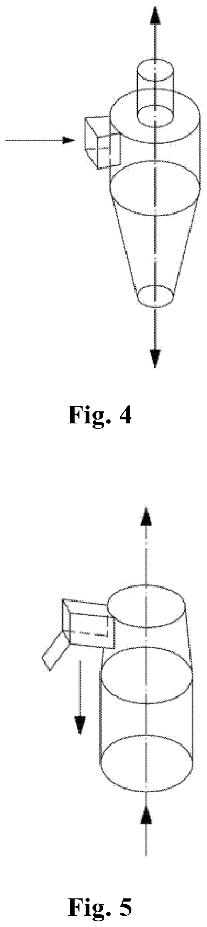

As shown in FIG. **4****,** in certain particularly preferred embodiments, the material inlet of the catalyst separator is located at the side of the catalyst separator, one material outlet is located at the bottom of the catalyst separator, and another material outlet is located at the upper portion of the catalyst separator.

As shown in FIG. 5, in some other particularly preferred embodiments, the material inlet of the catalyst separator is located at the bottom of the catalyst separator, one material outlet is located at the side of the catalyst separator, and another material outlet is located at the upper portion of the catalyst separator.

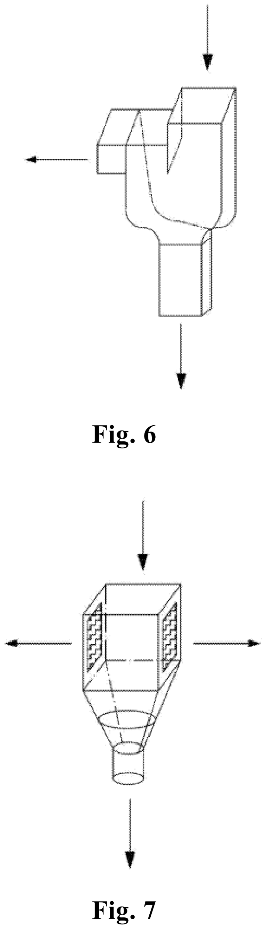

As shown in FIG. 6 and 7, in some other particularly preferred embodiments, the material inlet of the catalyst separator is located at the upper portion of the catalyst separator, one material outlet is located at the bottom of the catalyst separator, and another material outlet is located at the side of the catalyst separator. As shown in Figure 7, the material outlet on the side can be set in the form of a movable louver, the number and angle of which can be adjusted to control its flow rate.

According to the present application, when the catalyst separator is arranged outside the disengager, in certain further preferred embodiments, the catalyst separator is in the form of a two-stage separator connected in series, the inlet of the first-stage separator is connected to the catalyst outlet of the disengager. The first material outlet is arranged at the lower portion of the first-stage separator, and the light material outlet is arranged at its upper portion. The inlet of the second-stage separator is connected to the light material outlet of the first-stage separator. The second material outlet is arranged at the lower portion of the second-stage separator, and the residual reaction oil and gas outlet is arranged at its upper portion. Further preferably, the residual reaction oil and gas outlet of the second-stage separator is connected to the upper portion of the disengager through a pipeline.

According to the present application, when the catalyst separator is arranged inside the disengager, in certain further preferred embodiments, a settling zone partition is provided in the disengager to separate the first settling zone and the second settling zone. The first settling zone is located below the settling zone partition, and the second settling zone is located above the settling zone partition. The catalyst separator is fixed on the settling zone partition of the disengager so that its first material outlet is located in the first settling zone, and its second material outlet is located in the second settling zone.

In a preferred embodiment, the composite catalyst regenerator is provided with a regenerator partition separating the first regeneration zone and the second regeneration zone.

In certain further preferred embodiments, the regenerator partition is vertically arranged, and the first regeneration zone and the second regeneration zone are arranged on the left and right sides of the regenerator partition. Preferably, the upper portion of the first regeneration zone and the second regeneration zone are connected, and gas-solid separators are respectively arranged therein. The upper edge of the regenerator partition is higher than the solid outlet of each of the gas-solid separators.

In some further preferred embodiments, the regenerator partition is arranged horizontally, and the first regeneration zone and the second regeneration zone are arranged on the upper and lower sides of the regenerator partition. Preferably, the first regeneration zone is arranged below the second regeneration zone, and a first regenerated flue gas outlet is arranged on the regenerator partition so that the regenerated flue gas from the first regeneration zone can enter the second regeneration zone.

Certain specific embodiments of the fluidized catalytic conversion system of the first aspect of the present application are further described in detail below.

### The first type of embodiments

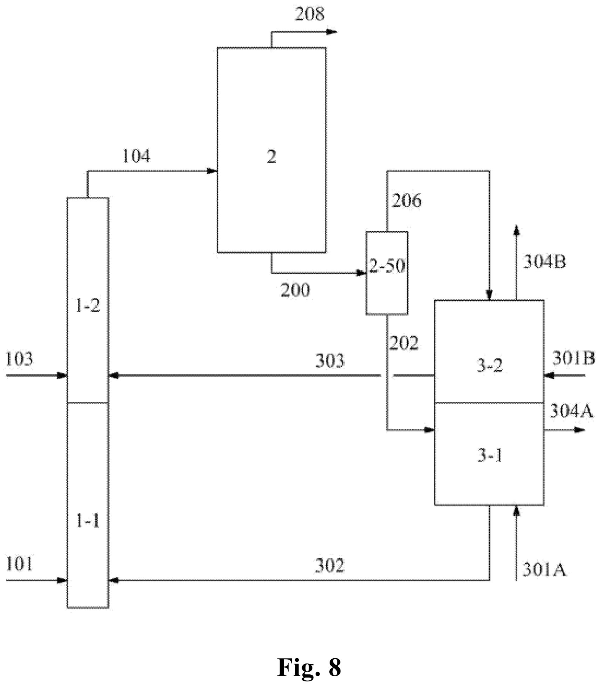

As shown in FIG. 8, the fluidized catalytic conversion system of the first embodiment includes a composite fluidized bed reactor having a first reaction zone 1-1 of an upflow bed type and a second reaction zone 1-2 of an upflow bed type, a disengager 2, a catalyst separator 2-50 and a regenerator. The first reaction zone 1-1 has a first feedstock oil inlet, a first catalyst inlet and a first oil catalyst mixture outlet. The second reaction zone 1-2 has an oil catalyst mixture inlet, a second catalyst inlet, a second oil catalyst mixture outlet and an optional second feedstock oil inlet. The first oil catalyst mixture outlet of the first reaction zone 1-1 is connected to the oil catalyst mixture inlet at the lower portion of the second reaction zone 1-2. The disengager 2 has an oil catalyst mixture inlet, an oil and gas mixture outlet and a catalyst outlet. The oil catalyst mixture inlet is connected to the second oil catalyst mixture outlet of the second reaction zone 1-2. The catalyst separator 2-50 is arranged outside the disengager 2 and has a material inlet, a first material outlet and a second material outlet. The material inlet is connected to the catalyst outlet of the disengager 2. A regenerator partition is provided in the regenerator to separate the regenerator into a first regeneration zone 3-1 and a second regeneration zone 3-2 arranged vertically. A first material outlet of the catalyst separator and the first regeneration zone 3-1 are connected for conveying a first spent catalyst. A second material outlet of the catalyst separator and the second regeneration zone 3-2 are connected for conveying a second spent catalyst. The first regeneration zone 3-1 and the first reaction zone 1-1 are connected for conveying a first regenerated catalyst. The second regeneration zone 3-2 and the second reaction zone 1-2 are connected for conveying a second regenerated catalyst.

In this embodiment, the first oil catalyst mixture outlet of the first reaction zone is connected to the oil catalyst mixture inlet at the lower portion of the second reaction zone, that is, the first reaction zone and the second reaction zone are connected in series, so that the materials (including the first catalyst, the reaction oil and gas and the fluidized medium) that have undergone the first catalytic conversion in the first reaction zone are allowed to enter the second reaction zone without separation, to contact the second catalyst for the second catalytic conversion reaction, that is, the first catalytic conversion reaction and the second catalytic conversion reaction can be carried out in relay. The disengager can perform gas-solid separation and stripping on the materials (including the first catalyst, the second catalyst, the reaction oil and gas and the fluidized medium) that have undergone the second catalytic conversion in the second reaction zone to obtain oil and gas products and mixed catalysts to be regenerated. The catalyst separator can separate the mixed catalysts to be regenerated, so that the one with a larger particle size and density of the first spent catalyst and the second spent catalyst basically enters the first regeneration zone, while the other with a smaller particle size and density basically enters the second regeneration zone. Subsequently, the two parts of the catalysts to be regenerated are regenerated in the first regeneration zone and the second regeneration zone respectively, thereby realizing the relatively independent regeneration of the first catalyst and the second catalyst. The regenerated first catalyst and the second catalyst are respectively returned to the first reaction zone and the second reaction zone to participate in the reaction again.

In operation, the first reaction zone has an upflow first catalyst. The second reaction zone has an upflow second catalyst and an upflow first catalyst. The particle size and density of the first catalyst are respectively greater than the particle size and density of the second catalyst. The catalyst separator separates the first catalyst from the second catalyst based on the difference between the particle size and density of the first catalyst and the particle size and density of the second catalyst. In the catalyst separator, a first material outlet for the first spent catalyst is located below a second material outlet for the second spent catalyst.

Optionally, part or all of the second reaction zone is disposed in the settling zone of the disengager 2. For example, a transverse pipe of the second reaction zone for conveying the second oil catalyst mixture 104 can be disposed in the settling zone of the disengager 2.

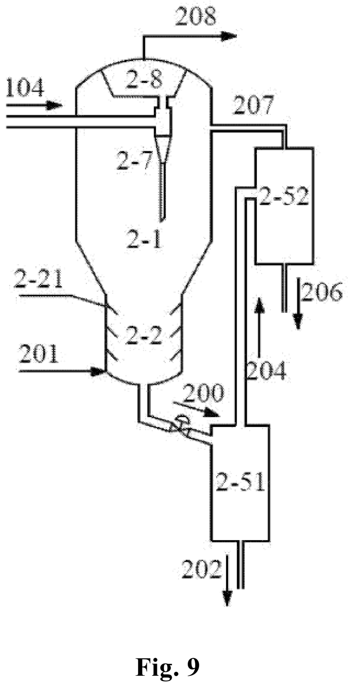

In certain preferred embodiments, as shown in FIG. 9, the disengager is externally connected to a two-stage catalyst separator, wherein the lower portion of the first- stage catalyst separator 2-51 has the first material outlet, and the upper portion of the first-stage catalyst separator has a light material outlet. The light material outlet is connected to the material inlet of the second-stage catalyst separator 2-52. The upper portion of the second-stage catalyst separator is provided with a residual reaction oil and gas outlet, and the lower portion of the second-stage catalyst separator is provided with a second material outlet.

In such a preferred embodiment, as shown in FIG. 9, the disengager has a settling zone 2-1 and a stripping section 2-2 connected to the bottom of the settling zone 2-1. An oil and gas product outlet is provided at the upper portion of the disengager. A gas-solid separation device is also provided inside the disengager. The second oil catalyst mixture 104 from the second reaction zone 1-2 is introduced into the fast separation device 2-7 of the disengager. The obtained reaction oil and gas 208 is introduced into the subsequent product separation unit (not shown in the figure) through the gas collecting chamber 2-8. The obtained mixed spent catalyst is introduced into the stripping section 2-2 for stripping. The mixed spent catalyst after stripping is introduced into the first-stage catalyst separator 2-51 and the second-stage catalyst separator 2-52. According to the particle size distribution and particle density of the catalyst, the mixed spent catalyst is divided into a first spent catalyst with a larger particle size and particle density and a second spent catalyst with a smaller particle size and particle density. The first spent catalyst is introduced into the first regeneration zone 3-1 through the first spent catalyst delivery pipe 202 for regeneration. The second spent catalyst is introduced into the second regeneration zone 3-2 through the second spent catalyst delivery pipe 206 for regeneration. The residual reaction oil and gas 207 in the catalyst separator returns to the upper portion of the disengager through the pipeline and is led out of the system together with the reaction oil and gas 208.

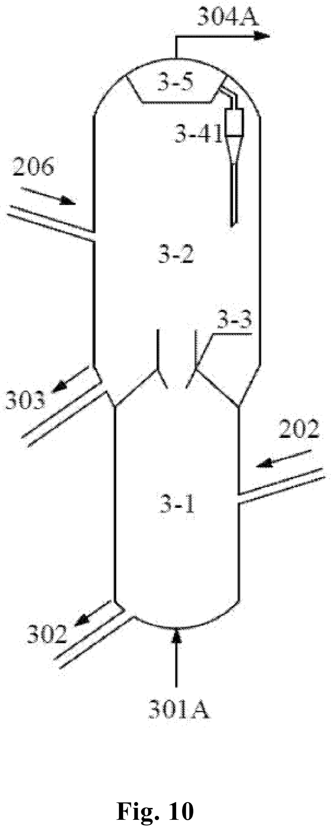

In certain preferred embodiments, as shown in FIG. 10, the first regeneration zone 3-1 and the second regeneration zone 3-2 are arranged in series up and down. The first regeneration zone 3-1 is located below the second regeneration zone 3-2. They are separated by a regenerator partition 3-3, wherein the regenerator partition is provided with a first regenerated flue gas outlet. The first spent catalyst from the catalyst separator is introduced into the first regeneration zone 3-1 through the first spent catalyst delivery pipe 202, contacts and reacts with the main air from the bottom of the first regeneration zone 3-1, for a first regeneration reaction to generate a first regenerated catalyst and a first regenerated flue gas. The generated first regenerated catalyst is introduced into the first reaction zone 1-1 through the first regeneration catalyst delivery pipe 302 for recycling. The first regenerated flue gas is introduced into the second regeneration zone 3-2 through the first regenerated flue gas outlet on the regenerator partition 3-3. The second spent catalyst from the catalyst separator is introduced into the second regeneration zone 3-2 through the second spent catalyst delivery pipe 206, and contacts and reacts with the first regenerated flue gas from the first regeneration zone 3-1 for second regeneration reaction to generate a second regenerated catalyst and a second regenerated flue gas. The generated second regenerated catalyst is introduced into the second reaction zone 1-2 through the second regenerated catalyst delivery pipe 303 for recycling. The second regenerated flue gas 304A is led out of the regenerator through the gas collecting chamber 3-5. The first regeneration adopts incomplete regeneration, and the second regeneration adopts complete regeneration. That is, the first regenerated flue gas obtained in the first regeneration zone 3-1 is introduced into the second regeneration zone 3-2 to undergo a regeneration reaction with the second regenerated catalyst.

In certain preferred embodiments, the fluidized catalytic conversion system is operated as follows: the first feedstock oil 101 is preheated to 180-340°C and then sprayed into the bottom of the first reaction zone 1-1 of the riser type through a nozzle, and contacts and reacts with the first catalyst introduced into the bottom of the first reaction zone 1-1 through the first regenerated catalyst delivery pipe 302, under the conditions of a reaction temperature of 520-620°C, preferably 540-600°C, a catalyst-oil ratio of 2-25, preferably 3-20; and a reaction time of 1-15 seconds, preferably 2-10 seconds. The resulting first oil catalyst mixture is introduced into the second reaction zone 1-2 of the riser type. After the second feedstock oil 103 is preheated to 100-150°C, it is sprayed into the bottom of the second reaction zone 1-2 through a nozzle, and contacts and reacts with the second catalyst introduced into the bottom of the second reaction zone 1-2 through the second regenerated catalyst delivery pipe 303 and the first oil catalyst mixture from the first reaction zone 1-1, under the conditions of a reaction temperature of 540-640°C, preferably 560-620°C, a catalyst-oil ratio of 3-30, preferably 5-25; and a reaction time of 1-10 seconds, preferably 2-8 seconds. The resulting oil catalyst mixture is introduced into a fast separator 2-7 of the disengager. The obtained reaction oil and gas 208 is led out of the device through the gas collecting chamber 2-8. The obtained mixed spent catalyst is introduced into the stripping section 2-2 for stripping. The mixed spent catalyst after stripping is introduced into the first-stage catalyst separator 2-51 and the second-stage catalyst separator 2-52. The first spent catalyst is introduced into the first regeneration zone 3-1 for regeneration through the first spent catalyst delivery pipe 202. The second spent catalyst is introduced into the second regeneration zone 3-2 for regeneration through the second spent catalyst delivery pipe 206. The regenerated first regenerated catalyst and the second regenerated catalyst are respectively returned to the bottom of the first reaction zone 1-1 and the second reaction zone 1-2 for recycling through the first regenerated catalyst delivery pipe 302 and the second regenerated catalyst delivery pipe 303.

### The second type of embodiments

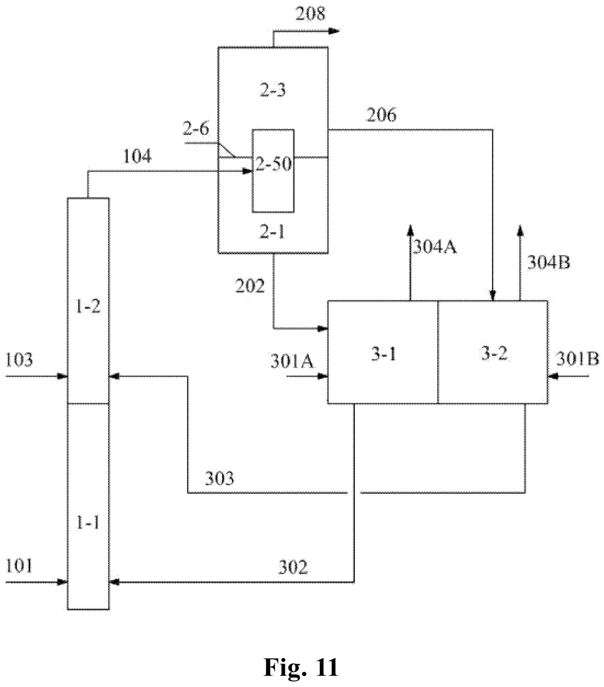

As shown in FIG. 11, the fluidized catalytic conversion system of the second embodiment includes a composite fluidized bed reactor having a first reaction zone 1-1 of an upflow bed type and a second reaction zone 1-2 of an upflow bed type, a disengager and a regenerator. The first reaction zone 1-1 has a first feedstock oil inlet, a first catalyst inlet and a first oil catalyst mixture outlet; the second reaction zone 1-2 has an oil catalyst mixture inlet, a second catalyst inlet, a second oil catalyst mixture outlet and an optional second feedstock oil inlet; the first oil catalyst mixture outlet of the first reaction zone 1-1 is connected to the oil catalyst mixture inlet at the lower portion of the second reaction zone 1-2. The disengager is provided with a settling zone partition 2-6 and a catalyst separator 2-50, wherein the settling zone partition 2-6 separates the settling zone of the disengager into a first settling zone 2-1 and a second settling zone 2-3. The first settling zone 2-1 is located below the settling zone partition 2-6, and the second settling zone 2-3 is located above the settling zone partition 2-6. The catalyst separator 2-50 has a material inlet connected to the second oil catalyst mixture outlet of the second reaction zone 1-2, a first material outlet opened in the first settling zone 2-1, and a second material outlet opened in the second settling zone 2-3. The regenerator is provided with a regenerator partition, which separates the regenerator into a first regeneration zone 3-1 and a second regeneration zone 3-2 arranged side by side on the left and right. A first spent catalyst is transported and connected between the first settling zone 2-1 and the first regeneration zone 3-1. A second spent catalyst is transported and connected between the second settling zone 2-3 and the second regeneration zone 3-2. A first regenerated catalyst is transported and connected between the first regeneration zone 3-1 and the first reaction zone 1-1. A second regenerated catalyst is transported and connected between the second regeneration zone 3-2 and the second reaction zone 1-2.

In this embodiment, the first oil catalyst mixture outlet of the first reaction zone is connected to the oil catalyst mixture inlet at the lower portion of the second reaction zone, that is, the first reaction zone and the second reaction zone are connected in series, so that the materials (including the first catalyst, the reaction oil and gas and the fluidized medium) that have undergone the first catalytic conversion in the first reaction zone are allowed to enter the second reaction zone without separation, to contact the second catalyst for the second catalytic conversion reaction. That is, the first catalytic conversion reaction and the second catalytic conversion reaction can be carried out in relay. The catalyst separator can separate the materials (including the first catalyst, the second catalyst, the reaction oil and gas and the fluidized medium) that have undergone the second catalytic conversion in the second reaction zone, so that the one of the first spent catalyst and the second spent catalyst, which has a larger particle size and density, basically enters the first settling zone, and the other, which has a smaller particle size and density, basically enters the second settling zone, together with the reaction oil and gas and the fluidized medium. Subsequently, the spent catalyst in the first settling zone is regenerated in the first regeneration zone, and the spent catalyst in the second settling zone is regenerated in the second regeneration zone, thereby realizing the relatively independent regeneration of the first catalyst and the second catalyst. The regenerated first catalyst and the second catalyst return to the first reaction zone and the second reaction zone respectively to participate in the reaction again.

In operation, the first reaction zone has an upflow first catalyst. The second reaction zone has an upflow second catalyst and an upflow first catalyst. The particle size and density of the first catalyst are respectively greater than the particle size and density of the second catalyst. The catalyst separator separates the first catalyst from the second catalyst based on the difference between the particle size and density of the first catalyst and the particle size and density of the second catalyst.

Optionally, part or all of the second reaction zone is disposed in the first settling zone or the second settling zone of the disengager. For example, the transverse pipe of the second reaction zone for conveying the second oil catalyst mixture 104 can be disposed in the second settling zone of the disengager.

Preferably, first and second stripping sections are provided which are connected to the bottom of the first settling zone and the bottom of the second settling zone respectively, and can be arranged inside or outside the disengager to extract the oil and gas products attached to the catalyst.

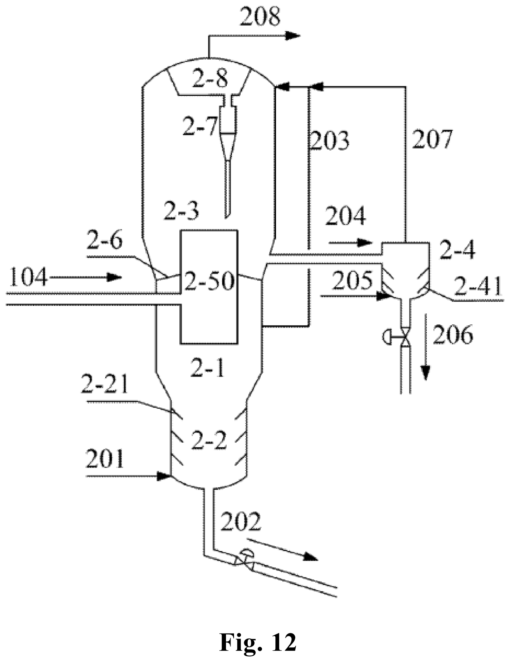

In certain preferred embodiments, as shown in FIG. 12, the catalyst separator 2-50 is fixed on the settling zone partition 2-6. The bottom of the first settling zone 2-1 is connected to the first stripping section 2-2. The first settling zone 2-1 is provided with a first stripping material outlet at the upper portion. The first stripping material outlet is connected to the pipeline 203. The bottom of the second settling zone 2-3 is connected to the second stripping section 2-4 provided outside the disengager. The second stripping material outlet is also provided at the upper portion of the second stripping section 2-4. The second stripping material outlet is connected to the pipeline 207.

In such a preferred embodiment, as shown in FIG. 12, the second oil catalyst mixture 104 from the second reaction zone is introduced into the catalyst separator 2-50. Based on the particle size distribution and particle density of the catalyst, the second oil catalyst mixture 104 is divided into a first part of catalyst with a larger particle size and particle density and a second part of catalyst with a smaller particle size and particle density. The first part of the catalyst is introduced into the first stripping section 2-2 via the first settling zone 2-1 for stripping to obtain a first spent catalyst. The first spent catalyst is introduced into the first regeneration zone 3-1 for regeneration through the first spent catalyst delivery pipe 202. The second part of the catalyst is introduced into the second stripping section 2-4 for stripping via the second settling zone 2-3 to obtain a second spent catalyst. The second spent catalyst is introduced into the second regeneration zone 3-2 for regeneration through the second spent catalyst delivery pipe 206. The first stripping material obtained in the first stripping section 2-2 and the second stripping material obtained in the second stripping section 2-4 are both introduced into the upper portion of the disengager and led out of the system together with the reaction oil and gas 208.

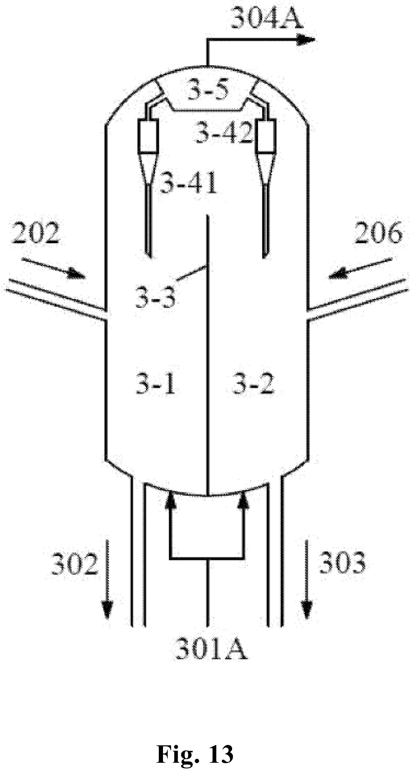

In certain preferred embodiments, as shown in Fig. 13, the first regeneration zone 3-1 and the second regeneration zone 3-2 are arranged side by side on the left and right. Optionally, the first regeneration zone 3-1 and the second regeneration zone 3-2 are connected in the upper portion. The first regeneration zone 3-1 and the second regeneration zone 3-2 are respectively provided with gas-solid separators 3-41 and 3-42. The upper edge of the regenerator partition 3-3 is higher than the solid outlets of the gas-solid separators 3-41 and 3-42.

In such a preferred embodiment, as shown in FIG. 13, the first spent catalyst from the first stripping section 2-2 is introduced into the first regeneration zone 3-1 through the first spent catalyst delivery pipe 202, contacts and reacts with the main air from the bottom of the first regeneration zone 3-1 for a regeneration reaction. The generated first regenerated catalyst is introduced into the first reaction zone 1-1 through the first regenerated catalyst delivery pipe 302 for recycling. The regenerated flue gas 304A is led out of the regenerator through the gas collecting chamber 3-5. The second spent catalyst from the second stripping section 2-4 is introduced into the second regeneration zone 3-2 through the second spent catalyst delivery pipe 206, contacts and reacts with the main air from the bottom of the second regeneration zone 3-2 for a regeneration reaction. The generated second regenerated catalyst is introduced into the second reaction zone 1-2 for recycling through the second regenerated catalyst delivery pipe 303. The regenerated flue gas 304 A is led out of the regenerator through the gas collecting chamber 3-5.

In a second aspect, the use of the fluidized catalytic conversion system of the present application for catalytic conversion of hydrocarbon oil, in particular, for catalytic conversion of heavy feedstock oil to produce light olefins is provided.

In a third aspect, a method for catalytic conversion of hydrocarbon oil, especially heavy feedstock oil, using the fluidized catalytic conversion system of the present application is provided, comprising the following steps:
1) contacting the hydrocarbon oil feedstock with a first catalyst in a first fluidized bed reactor or a first reaction zone of a composite fluidized bed reactor of a reaction unit of the fluidized catalytic conversion system, to perform a first catalytic conversion reaction to obtain a first oil catalyst mixture;
2) contacting the first oil catalyst mixture with a second catalyst in a second fluidized bed reactor or a second reaction zone of a composite fluidized bed reactor of a reaction unit of the fluidized catalytic conversion system, to perform a second catalytic conversion reaction to obtain a second oil catalyst mixture;
3) separating the second oil catalyst mixture in a catalyst separation unit of the fluidized catalytic conversion system to obtain an oil and gas product, a first spent catalyst, and a second spent catalyst;
4) regenerating the first spent catalyst in the first catalyst regenerator or the first regeneration zone of the composite catalyst regenerator of the catalyst regeneration unit of the fluidized catalytic conversion system, and returning the obtained first regenerated catalyst to step 1) as the first catalyst; and
5) regenerating the second spent catalyst in the second catalyst regenerator or the second regeneration zone of the composite catalyst regenerator of the catalyst regeneration unit of the fluidized catalytic conversion system, and returning the obtained second regenerated catalyst to step 2) as the second catalyst.

In a preferred embodiment, the particle size and density of the first catalyst are respectively greater than the particle size and density of the second catalyst.

In a preferred embodiment, the first catalyst contains 60-100 mass%, preferably 80-100 mass% of heavy oil catalyst and 0-40 mass%, preferably 0-20 mass% of light oil catalyst, and the second catalyst contains 0-40 mass%, preferably 0-20 mass% of heavy oil catalyst and 60-100 mass%, preferably 80-100 mass% of light oil catalyst.

In a further preferred embodiment, based on the total weight of the heavy oil catalyst, the heavy oil catalyst comprises:
10-80%, preferably 30-60% of modified or unmodified Y-type molecular sieve;
0-40%, preferably 0-20%, of modified or unmodified βmolecular sieve;
0-40%, preferably 0-20 % of modified or unmodified ZSM-5 molecular sieve;
10-80%, preferably 15-60% of clay;
10-30%, preferably 10-20% of a binder; and
0-40 %, preferably 0-20% of a heat carrier, wherein the heat carrier is selected from SiO₂, MgO, CaO, BaO and MnO₂, or any mixture thereof.

In a further preferred embodiment, in the heavy oil catalyst, the modified or unmodified Y molecular sieve is one or more selected from HY, USY, REUSY, REY, REHY, DASY, and REDASY, or a Y-type molecular sieve obtained by treatment with various metal oxides. The modification method of the Y molecular sieve may include methods well known to those skilled in the art such as impregnation, ion exchange, and sol-gel method. The modified or unmodified ZSM-5 molecular sieve may be one or more selected from ZRP zeolite, phosphorus-containing ZRP zeolite, rare earth-containing ZRP zeolite, phosphorus- and rare earth-containing ZRP zeolite, phosphorus- and alkaline earth metal-containing ZRP zeolite, and phosphorus- and transition metal-containing ZRP zeolite, preferably phosphorus- and rare earth-containing ZRP zeolite. The modification method of the ZSM-5 molecular sieve may include impregnation, ion exchange, and sol-gel method and other methods well known to those skilled in the art. The modified βmolecular sieve may be a βmolecular sieve modified by phosphorus and a transition metal M, wherein M may be one or more selected from Fe, Co, Ni, Cu, Mn, Zn and Sn. The βmolecular sieve modified by phosphorus and a transition metal M may be prepared by various methods, such as introducing phosphorus and the transition metal M during the synthesis of the βmolecular sieve, or introducing phosphorus and the transition metal M by steps including ammonium exchange, phosphorus modification, modification of the transition metal M and calcination treatment after the synthesis of the βmolecular sieve. The clay may be selected from various clays that can be used as catalyst components, such as kaolin, montmorillonite, bentonite, etc. The binder may be one selected from silica sol, alumina sol and pseudo-boehmite, or a mixture of two or three selected from silica sol, alumina sol and pseudo-boehmite, wherein the preferred binder is a bialuminum binder of alumina sol and pseudo-boehmite.

In a preferred embodiment, based on the total weight of the light oil catalyst, the light oil catalyst comprises:
10-60%, preferably 20-50% of modified or unmodified ZSM-5 molecular sieve;
0-40%, preferably 0-20%, of modified or unmodified Y molecular sieve;
0-40%, preferably 0-20% of modified or unmodified β-structured molecular sieve;
10-80%, preferably 20-70% of clay;
10-30%, preferably 10-20% of a binder; and
0-40%, preferably 0-20 % of a heat carrier, wherein the heat carrier is selected from SiO₂, MgO, CaO, BaO and MnO₂, or any mixture thereof.

In a further preferred embodiment, in the light oil catalyst, the modified or unmodified ZSM -5 molecular sieve can be one or more selected from ZRP zeolite, phosphorus-containing ZRP zeolite, rare earth-containing ZRP zeolite, phosphorus- and rare earth-containing ZRP zeolite, phosphorus- and alkaline earth metal-containing ZRP zeolite, and phosphorus- and transition metal-containing ZRP zeolite, preferably phosphorus- and rare earth-containing ZRP zeolite. The modification method of the ZSM-5 molecular sieve includes impregnation method, ion exchange method, sol-gel method and other methods well known to those skilled in the art. The modified or unmodified Y molecular sieve is one or more selected from HY, USY, REUSY, REY, REHY, DASY, REDASY, or a Y-type molecular sieve obtained by treatment with various metal oxides. The modification method of the Y molecular sieve includes impregnation method, ion exchange method, sol-gel method and other methods well known to those skilled in the art. The modified βmolecular sieve may be a βmolecular sieve modified by phosphorus and a transition metal M, wherein M is one or more selected from Fe, Co, Ni, Cu, Mn, Zn and Sn. The βmolecular sieve modified by phosphorus and a transition metal M may be prepared by various methods, such as introducing phosphorus and the transition metal M during the synthesis of the βmolecular sieve, or introducing phosphorus and the transition metal M by steps including ammonium exchange, phosphorus modification, modification of the transition metal M and calcination treatment after the synthesis of the β molecular sieve. The clay may be selected from various clays that can be used as catalyst components, such as kaolin, montmorillonite, bentonite, etc. The binder may be one or a mixture of two or three selected from silica sol, alumina sol and pseudo-boehmite, wherein the preferred binder is a bialuminum binder of alumina sol and pseudo-boehmite.

In a further preferred embodiment, the heavy oil catalyst has a particle size range of 60-250 µm, preferably 80-200 µm, and a particle density of 1200-1600 kg/m³, preferably 1300-1500 kg/m³, and the light oil catalyst has a particle size range of 10-100 µm, preferably 30-80 µm, and a particle density of 800-1200 kg/m³, preferably 900-1100 kg/m³.

In certain preferred embodiments, the first fluidized bed reactor or the first reaction zone of the composite fluidized bed reactor is of the type of riser reactor, wherein the reaction conditions include: reaction temperature of 520-620°C, preferably 540-600°C; catalyst-oil ratio of 2-25, preferably 3-20; reaction time of 1-15 seconds, preferably 2-10 seconds; and/or, the second fluidized bed reactor or the second reaction zone of the composite fluidized bed reactor is of the type of dense phase bed reactor, wherein the reaction conditions include: reaction temperature of 540-640°C, preferably 560-620°C, catalyst distribution density of 20-300 kg/m³ , preferably 100-200 kg/m³ , space velocity of 2-15 h⁻¹, preferably 5-10 h⁻¹; oil and gas residence time of 0.2-8 seconds, preferably 1-4 seconds.

In other preferred embodiments, the first fluidized bed reactor or the first reaction zone of the composite fluidized bed reactor is of the type of a fast bed reactor, wherein the reaction conditions include: reaction temperature of 520-620°C, preferably 540-600°C; agent-oil ratio of 2-25, preferably 3-20; reaction time of 1-15 seconds, preferably 2-10 seconds; and/or, the second fluidized bed reactor or the second reaction zone of the composite fluidized bed reactor is of the type of a fast bed reactor, wherein the reaction conditions include: reaction temperature of 540-640°C, preferably 560-620 °C, agent-oil ratio of 3-30, preferably 5-25; reaction time of 1-10 seconds, preferably 2-8 seconds.

In a preferred embodiment, the first catalyst regenerator or the first regeneration zone of the composite catalyst regenerator adopts incomplete regeneration. The second catalyst regenerator or the second regeneration zone of the composite catalyst regenerator adopts complete regeneration. The incompletely regenerated flue gas generated in the first catalyst regenerator or the first regeneration zone of the composite catalyst regenerator is introduced into the second catalyst regenerator or the second regeneration zone of the composite catalyst regenerator to continue the regeneration reaction.

In a preferred embodiment, the regeneration conditions in the first catalyst regenerator or the first regeneration zone of the composite catalyst regenerator include: regeneration temperature of 640-700°C, preferably 660-680°C, catalyst distribution density of 50-400 kg/m³, preferably 100-300 kg/m³, main air residence time of 0.5-20s, preferably 2-10s.

In a preferred embodiment, the regeneration conditions in the second catalyst regenerator or the second regeneration zone of the composite catalyst regenerator include: regeneration temperature of 670-730°C, preferably 690-710°C, catalyst distribution density of 30-350 kg/m³, preferably 80-250 kg/m³, main air residence time of 0.5-15s, preferably 2-10s.

In a preferred embodiment, the heavy feedstock oil is selected from vacuum gas oil, atmospheric residue oil, vacuum residue oil, coker gas oil, deasphalted oil, furfural refining raffinate oil, coal liquefaction oil, asphalt, shale oil, Fischer-Tropsch synthesis distillate oil, animal oil, vegetable oil, crude oil, biomass oil, or a mixture thereof.

The following is a further detailed description of certain specific implementations of the catalytic conversion method of the third aspect of the present application.

### The First Type of Embodiments

The catalytic conversion method of the first specific embodiment comprises the following steps:
1) contacting the heavy feedstock oil with a first catalyst in a first reactor/reaction zone of a riser reactor type to perform a first catalytic conversion reaction to obtain a first oil catalyst mixture;
2) introducing the first oil catalyst mixture into a second reactor/reaction zone of a dense phase bed reactor type to contact with a second catalyst to perform a second catalytic conversion reaction to obtain a second oil catalyst mixture;
3) subjecting the second oil catalyst mixture to gas-solid separation and steam stripping in a disengager to obtain an oil and gas product and a mixed spent catalyst;
4) introducing the mixed spent catalyst into a catalyst separator outside the disengager for separation, to obtain a first spent catalyst and a second spent catalyst, wherein the catalyst separator comprises a first-stage catalyst separator and a second-stage catalyst separator;
5) performing a first regeneration on the first spent catalyst and returning it to step 1) as the first catalyst to participate in the first catalytic conversion reaction, and performing a second regeneration on the second spent catalyst and returning it to step 2) as the second catalyst to participate in the second catalytic conversion reaction.

In this type of embodiments, the materials that have undergone the first catalytic conversion in the first reactor/reaction zone (including the first catalyst, reaction oil and gas, and fluidizing medium) all enter the second reactor /reaction zone without separation, contact with the second catalyst, and subject to a second catalytic conversion reaction. That is, the first catalytic conversion reaction and the second catalytic conversion reaction can be carried out in relay.

In certain preferred embodiments, the reaction conditions in the first reactor/reaction zone include: a reaction temperature of 520-620°C, preferably 540-600°C; a catalyst-oil ratio of 2-25, preferably 3-20; and a reaction time of 1-15 seconds, preferably 2-10 seconds. Optionally, the heavy feedstock oil can be preheated to 180-340 °C and then sprayed into the first reactor/reaction zone through a nozzle.

In certain preferred embodiments, the reaction conditions in the second reactor/reaction zone include: reaction temperature of 540-640°C, preferably 560-620°C, catalyst distribution density of 20-300 kg/m³, preferably 100-200 kg/m³, space velocity of 2-15h⁻¹, preferably 5-10h⁻¹; oil and gas residence time of 0.2-8 seconds, preferably 1-4 seconds.

Optionally, the catalytic conversion method further comprises introducing a pre-lifting gas into the bottom of the first reactor/reaction zone, wherein the pre-lifting gas may be one or more selected from water vapor, nitrogen, and dry gas, preferably water vapor.

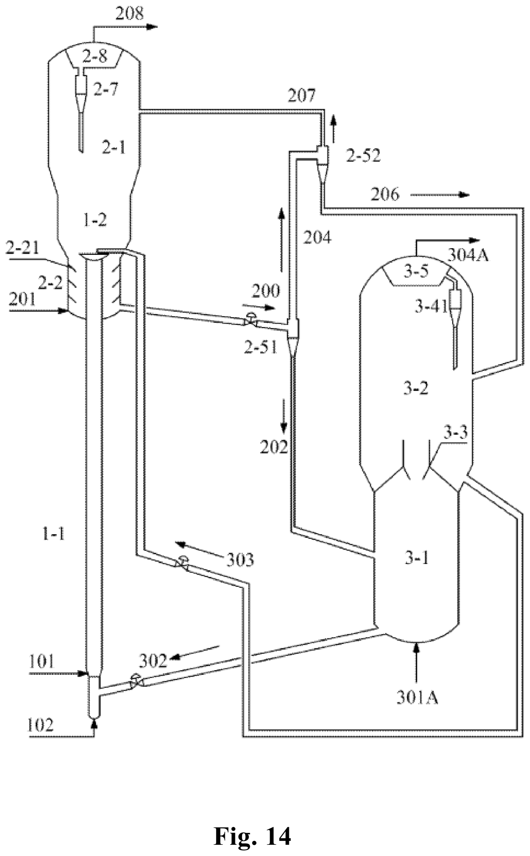

In certain preferred embodiments, as shown in FIG. 14, the fluidized catalytic conversion system comprises a composite fluidized bed reactor having a first reaction zone 1-1 in the form of a riser reactor and a second reaction zone 1-2 in the form of a dense phase bed reactor, a disengager having a settling zone 2-1, a stripping section 2-2 and a cyclone separator 2-7, and a composite catalyst regenerator having a first regeneration zone 3-1 and a second regeneration zone 3-2. The outlet of the first reaction zone 1-1 is connected to the inlet of the second reaction zone 1-2. The second reaction zone 1-2 is arranged in the settling zone 2-1 of the disengager and is located at the lower portion of the settling zone. The bottom of the second reaction zone 1-2 is connected to the stripping section 2-2. The inlet of the cyclone separator 2-7 is located at the upper portion of the disengager. The catalyst outlet position of the cyclone separator 2-7 enables the catalyst therein to enter the stripping section 2-2. The oil and gas outlet of the cyclone separator 2-7 is connected to the oil and gas separation system. The catalytic conversion system also includes a first-stage catalyst separator 2-51 and a second- stage catalyst separator 2-52. The inlet of the first-stage catalyst separator 2-51 is connected to the stripping section 2-2 through a spent catalyst delivery pipe 200. The first material outlet of the first-stage catalyst separator 2-51 is connected to the first regeneration zone 3-1 through a first spent catalyst delivery pipe 202. The light material outlet of the first-stage catalyst separator 2-51 is connected to the inlet of the second- stage catalyst separator 2-52 through a light material delivery pipe 204. The second material outlet of the second-stage catalyst separator 2-52 is connected to the second regeneration zone 3-2 through a second spent catalyst delivery pipe 206. The residual oil and gas outlet of the second stage catalyst separator 2-52 is connected to the upper portion of the disengager through an oil and gas delivery pipe 207. The delivery speed of the catalyst can be adjusted by the valve on the catalyst delivery pipe. The first regeneration zone 3-1 and the second regeneration zone 3-2 of the composite catalyst regenerator are arranged in series, and a regenerator partition 3-3 is set between them. The incompletely regenerated flue gas generated in the first regeneration zone 3-1 is introduced into the second regeneration zone 3-2 to continue the regeneration reaction. The obtained regenerated flue gas 304A is separated from the catalyst carried by the cyclone separator 3-41 and then enters the subsequent flue gas treatment system through the gas collecting chamber 3-5 outlet device. The first regenerated catalyst outlet of the first regeneration zone 3-1 is connected to the bottom of the first reaction zone 1-1 through the first regenerated catalyst delivery pipe 302. The second regenerated catalyst outlet of the second regeneration zone 3-2 is connected to the bottom of the second reaction zone 1-2 through the second regenerated catalyst delivery pipe 303. The delivery speed of the catalyst can be adjusted by the valve on the catalyst delivery pipe.

In certain preferred embodiments , in the catalytic conversion method , the heavy feedstock oil 101 is preheated to 180-340° C. and then sprayed into the first reaction zone 1-1 through a nozzle, and contacts and reacts with the first catalyst introduced into the bottom of the first reaction zone 1-1 through the first regenerated catalyst delivery pipe 302 under the conditions of a reaction temperature of 520-620° C, preferably 540-600° C; a catalyst-oil ratio of 2-25, preferably 3-20; and a reaction time of 1-15 seconds, preferably 2-10 seconds. The first oil catalyst mixture after the reaction is introduced into the second reaction zone 1-2, and contacts and reacts with the second catalyst introduced into the second reaction zone 1-2 through the second regenerated catalyst delivery pipe 303 under the conditions of reaction temperature of 540-640°C, preferably 560-620°C, catalyst density of 20-300 kg/m³, preferably 100-200 kg/m³, space velocity of 2-15h⁻¹ , preferably 5-10h⁻¹, and oil and gas residence time of 0.2-8 seconds, preferably 1-4 seconds. The second oil catalyst mixture after the reaction is separated by a cyclone separator 2-7, and the obtained reaction oil and gas 208 is collected by a gas collecting chamber 2-8 and introduced into a subsequent product separation system. The obtained spent catalyst is introduced into the stripping section 2-2 for stripping. The mixed spent catalyst after stripping is introduced into the first- stage catalyst separator 2-51 through the spent catalyst delivery pipe 200. The separated first spent catalyst is introduced into the first regeneration zone 3-1 through the first spent catalyst delivery pipe 202. Under the conditions of regeneration temperature of 640-700°C, preferably 660-680°C, catalyst density of 50-400 kg/m³, preferably 100-300 kg/m³, and main air residence time of 0.5-20s, preferably 2-10s, the separated first spent catalyst contacts with the main air 301A introduced into the first regeneration zone 3-1 for regeneration reaction. Incomplete regeneration is carried out in the first reaction zone 3-1. The obtained incompletely regenerated flue gas is introduced into the second reaction zone 3-2 through the regeneration baffle 3-3. The obtained first regenerated catalyst is introduced into the bottom of the first reaction zone 1-1 through the first regenerated catalyst delivery pipe 302 for recycling. The light material separated by the first-stage catalyst separator 2-51 is introduced into the second-stage catalyst separator 2-52 through the gas-solid mixture conveying pipe 204. The separated second regenerated catalyst is introduced into the second regeneration zone 3-2 through the second regenerated catalyst conveying pipe 206, and contacts with the incompletely regenerated flue gas from the first regeneration zone 3-1 for a regeneration reaction under the conditions of a regeneration temperature of 670-730°C, preferably 690-710°C, a catalyst density of 30-350 kg/m³, preferably 80-250 kg/m³, and a main air residence time of 0.5-15s, preferably 2-10s. A complete regeneration is carried out in the second reaction zone 3-2. The obtained completely regenerated flue gas 304A is separated from the catalyst carried by the cyclone separator 3-41, and then collected by the gas collecting chamber 3-5 and introduced into the regenerated flue gas treatment system. The obtained second regenerated catalyst is introduced into the bottom of the second reaction zone 1-2 through the second regenerated catalyst conveying pipe 303 for recycling. The residual oil and gas separated by the second-stage catalyst separator 2-52 is introduced into the disengager through the oil and gas delivery pipe 207, and is introduced into the subsequent product separation system after mixing with the reaction oil and gas 208. The reaction oil and gas 208 enters the subsequent product separation system. In the product separation system, the catalytic cracking products are separated into products such as dry gas, cracked gas, gasoline, light oil and oil slurry. The cracked gas is subjected to a subsequent product separation and refining to obtain polymerization-grade propylene products and a mixture of C4-C8 hydrocarbons. The stripping steam in the stripping section 2-2 can directly enter the settling area 2-1, and after being separated by the cyclone separator 2-7 together with other oil and gas, it is led out of the disengager by the reaction oil and gas 208.

### The second type of embodiments

The catalytic conversion method of the second type of embodiments comprises the following steps:
1) contacting the heavy feedstock oil with a first catalyst in a first reactor/reaction zone of a fast bed reactor type, to perform a first catalytic conversion reaction, to obtain a first oil catalyst mixture;
2) introducing the first oil catalyst mixture into a second reactor/reaction zone of a fast bed reactor type, to contact with a second catalyst to perform a second catalytic conversion reaction, to obtain a second oil catalyst mixture;
3) introducing the second oil catalyst mixture into a catalyst separation device inside a disengager for separation to obtain a first spent catalyst and a third oil catalyst mixture;
4) separating and stripping the third oil mixture to obtain reaction oil and gas, stripping products and a second spent catalyst;
5) subjecting the first spent catalyst to a first stripping and a first regeneration, then returning it to step 1) as the first catalyst to participate in the first catalytic conversion reaction, and subjecting the second spent catalyst to a second stripping and a second regeneration, then returning it to step 2) as the second catalyst to participate in the second catalytic conversion reaction.

In this type of embodiments, the materials that have undergone the first catalytic conversion in the first reactor/reaction zone (including the first catalyst, reaction oil and gas, and fluidizing medium) all enter the second reactor/reaction zone without separation, contact with the second catalyst for a second catalytic conversion reaction. That is, the first catalytic conversion reaction and the second catalytic conversion reaction can be carried out in relay.

In certain preferred embodiments, the reaction conditions in the first reactor/reaction zone include: a reaction temperature of 520-620°C, preferably 540-600°C; a catalyst-oil ratio of 2-25, preferably 3-20; a reaction time of 1-15 seconds, preferably 2-10 seconds. Optionally, the heavy feedstock oil can be preheated to 180-340°C and then sprayed into the first reactor/reaction zone through a nozzle.

In certain preferred embodiments, the reaction conditions in the second reactor/reaction zone include: reaction temperature of 540-640°C, preferably 560-620°C, catalyst-oil ratio of 3-30 , preferably 5-25; reaction time of 1-10 seconds, preferably 2-8 seconds.

Optionally, the catalytic conversion method further comprises introducing a pre-lifting gas into the bottom of the first reactor/reaction zone, wherein the pre-lifting gas may be one or more selected from water vapor, nitrogen, and dry gas, preferably water vapor.

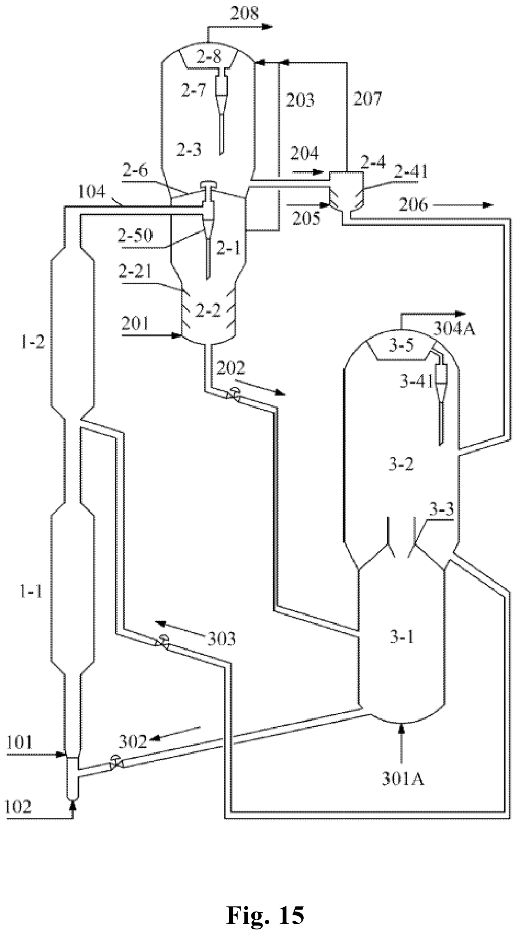

In certain preferred embodiments, as shown in FIG. 15, the fluidized catalytic conversion system comprises a composite fluidized bed reactor having a first reaction zone 1-1 in the form of a fast bed reactor and a second reaction zone 1-2 in the form of a fast bed reactor, a disengager having a first settling zone 2-1 and a second settling zone 2-3, and a composite catalyst regenerator having a first regeneration zone 3-1 and a second regeneration zone 3-2. The outlet of the first reaction zone 1-1 is connected to the inlet of the second reaction zone 1-2, and the outlet of the second reaction zone 1-2 is connected to the catalyst separator 2-50 inside the disengager. The catalyst separator 2-50 is located inside the disengager. The disengager partition 2-6 separates the first settling zone 2-1 and the second settling zone 2-3. The first material outlet of the catalyst separator 2-50 is located in the first settling zone 2-1. The second material outlet is located in the second settling zone 2-3. A cyclone separator 2-7 is also provided inside the disengager for separating the catalyst entrained in the reaction oil and gas. The inlet of the cyclone separator 2-7 is located at the upper portion of the second settling zone 2-3. The catalyst outlet position of the cyclone separator 2-7 enables the catalyst therein to enter the second settling zone 2-3. The oil and gas outlet of the cyclone separator 2-7 is connected to the oil and gas separation system. The obtained reaction oil and gas 208 is led out of the device. The bottom of the first settling zone 2-1 is connected to the first stripping section 2-2, so that the first spent catalyst is introduced into the first regeneration zone 3-1 for regeneration through the first spent catalyst delivery pipe 202 after stripping in the first stripping section 2-2. The bottom of the second settling zone 2-3 is connected to the second stripping section 2-4, so that the second spent catalyst is introduced into the second regeneration zone 3-2 for regeneration through the second spent catalyst delivery pipe 206 after stripping in the second stripping section 2-4. The first regeneration zone 3-1 and the second regeneration zone 3-2 of the composite catalyst regenerator are arranged in series. A regenerator partition 3-3 is arranged between the them, on which a first regenerated flue gas outlet is arranged to introduce the incompletely regenerated flue gas generated in the first regeneration zone 3-1 into the second regeneration zone 3-2 for continued regeneration reaction. The obtained regenerated flue gas 304 A is separated from the catalyst carried by the cyclone separator 3-41 and then enters the subsequent flue gas treatment system through the gas collecting chamber 3-5 outlet device. The first regeneration catalyst outlet of the first regeneration zone 3-1 is connected to the bottom of the first reaction zone 1-1 through the first regeneration catalyst delivery pipe 302. The second regeneration catalyst outlet of the second regeneration zone 3-2 is connected to the bottom of the second reaction zone 1-2 through the second regeneration catalyst delivery pipe 303. The delivery speed of the catalyst can be adjusted by the valve on the catalyst delivery pipe.

In certain preferred embodiments , in the catalytic conversion method, the heavy feedstock oil 101 is preheated to 180-340 °C and then sprayed into the first reaction zone 1-1 through a nozzle, and contacts and reacts with the first catalyst introduced into the bottom of the first reaction zone 1-1 through the first regenerated catalyst delivery pipe 302 under the conditions of a reaction temperature of 520-620°C, preferably 540-600°C; a catalyst-oil ratio of 2-25, preferably 3-20; and a reaction time of 1-15 seconds, preferably 2-10 seconds. The first oil catalyst mixture after the reaction is introduced into the second reaction zone 1-2 , and contacts and reacts with the second catalyst introduced into the second reaction zone 1-2 through the second regenerated catalyst delivery pipe 303 under the conditions of a reaction temperature of 540-640°C, preferably 560-620°C, agent-oil ratio of 3-30, preferably 5-25; reaction time of 1-10 seconds, preferably 2-8 seconds. The second oil catalyst mixture after the reaction is introduced into the catalyst separator 2-50, and is separated into the first spent catalyst and the third oil catalyst mixture. The first spent catalyst is introduced into the first stripping section 2-2 through the first settling zone 2-1 for stripping. The first spent catalyst after stripping is introduced into the first regeneration zone 3-1 through the first spent catalyst delivery pipe 202 for regeneration. The regenerated first catalyst is introduced into the bottom of the first reaction zone 1-1 through the first catalyst delivery pipe 302. The third oil catalyst mixture is separated in the cyclone separator 2-7 in the second settling zone 2-3 to obtain oil and gas products and the second regenerated catalyst. The second spent catalyst is introduced into the second stripping section 2-4 through the second settling zone 2-3 for stripping. The stripped second spent catalyst is introduced into the second regeneration zone 3-2 through the second spent catalyst delivery pipe 206 for regeneration. The regenerated second catalyst is introduced into the bottom of the second reaction zone 1-2 through the second catalyst delivery pipe 303. The main air 301 A is introduced into the first regeneration zone 3-1, and contacts with the first spent catalyst introduced into the first regeneration zone 3-1 for regeneration reaction, under the conditions of a regeneration temperature of 640-700°C, preferably 660-680°C, a catalyst density of 50-400 kg/m³, preferably 100-300 kg/m³, and a main air residence time of 0.5-20s, preferably 2-10s. Incomplete regeneration is performed in the first regeneration zone 3-1. The obtained incompletely regenerated flue gas is introduced into the second regeneration zone 3-2 through the regenerator partition 3-3, and contacts and performs a regeneration reaction with the second spent catalyst introduced into the second regeneration zone 3-2, under the conditions of a regeneration temperature of 670-730°C, preferably 690-710°C, a catalyst density of 30-350 kg/m³, preferably 80-250 kg/m³, and a main wind residence time of 0.5-15s, preferably 2-10s. A completely regenerated is performed in the second regeneration zone 3-2. The obtained completely regenerated flue gas 304A is separated from the carried catalyst by the cyclone separator 3-41, and then collected by the gas collecting chamber 3-5 and introduced into the regenerated flue gas treatment system. The reaction oil and gas 208 enters the subsequent product separation system. In the product separation system, the catalytic cracking products are separated into products such as dry gas, cracked gas, gasoline, light oil and oil slurry. The cracked gas is subjected to a subsequent product separation and refining to obtain polymerization-grade ethylene products and propylene products and a mixture of C4-C8 hydrocarbons. The stripping steam and residual oil and gas in the first stripping section 2-2 and the second stripping section 2-4 can directly enter the second settling zone 2-3. After being separated from other oil and gas by the cyclone separator 2-7, the stripping steam and residual oil and gas are led out of the disengager by the reaction oil and gas 208.

In certain preferred embodiments, the present application provides the following technical solutions:
1. A catalytic conversion system with a reactor and a catalyst separator in series, wherein the catalytic conversion system comprises a first upflow bed reactor, a second upflow bed reactor, a disengager, a catalyst separator and a regenerator; the first upflow bed reactor has a first catalyst inlet and a first oil catalyst mixture outlet; the second upflow bed reactor has a second catalyst inlet and a second oil catalyst mixture outlet; the first oil catalyst mixture outlet is connected to the lower portion of the second upflow bed reactor;
   the disengager has an oil catalyst mixture inlet and a mixed spent catalyst outlet, and the oil catalyst mixture inlet is connected to the second oil catalyst mixture outlet;
   the catalyst separator has a material inlet connected to the mixed spent catalyst outlet, a first spent catalyst outlet and a second spent catalyst outlet;
   a regenerator partition is provided in the regenerator, and the regenerator partition separates the regenerator into a first regeneration zone and a second regeneration zone; a first spent catalyst is transported and connected between the first spent catalyst outlet and the first regeneration zone; a second spent catalyst is transported and connected between the second spent catalyst outlet and the second regeneration zone; a first regenerated catalyst is transported and connected between the first regeneration zone and the first upward bed reactor; and a second regenerated catalyst is transported and connected between the second regeneration zone and the second upward bed reactor.
2. The catalytic conversion system according to item 1, wherein the first upflow bed reactor has an upward first catalyst; the second upflow bed reactor has an upward second catalyst and an upward first catalyst; the particle size and density of the first catalyst are greater than the particle size and density of the second catalyst; the outlet of the first spent catalyst is located below the outlet of the second spent catalyst.
3. The catalytic conversion system according to item 1 or 2, wherein a first feedstock oil inlet and a fluidized medium inlet are also provided at the lower portion of the first upflow bed reactor; a second feedstock oil inlet is also provided at the lower portion of the second upflow bed reactor.
4. The catalytic conversion system according to item 1 or 2, wherein the first upflow bed reactor and the second upflow bed reactor are each independently a riser reactor, a fast bed reactor, or a fluidized bed reactor.
5. The catalytic conversion system according to item 1 or 2, wherein the catalyst separator is arranged outside the disengager, and part or all of the second upflow bed reactor is arranged in the disengager.
6. The catalytic conversion system according to item 1 or 2, wherein the catalyst separator is one or a combination of multiple selected from a cyclone-type fast separator, a tri-lobe fast separator, a ballistic fast separator, a U-tube separator, and a wall-cutting fast separator, preferably a cyclone-type fast separator;
   the number of the catalyst separators connected to the outside of each disengager is one or more, and the multiple catalyst separators are connected in series and/or in parallel.
7. The catalytic conversion system according to item 6, wherein the disengager is externally connected to a first stage catalyst separator, the lower portion of the first stage catalyst separator is provided with a first spent catalyst outlet, and the upper portion of the first stage catalyst separator is provided with a light material outlet; the light material outlet is connected to a material inlet of a second stage catalyst separator; the upper portion of the second stage catalyst separator is provided with a residual reaction oil and gas outlet, and the lower portion of the second stage catalyst separator is provided with a second spent catalyst outlet.
8. The catalytic conversion system according to item 1 or 2, wherein a stripper is provided at the lower portion of the disengager; an oil and gas product outlet is provided at the upper portion of the disengager; and a gas-solid separation device is also provided inside the disengager.
9. The catalytic conversion system according to item 1 or 2, wherein the first regeneration zone and the second regeneration zone are arranged in series up and down and the first regeneration zone is located below the second regeneration zone.
10. The catalytic conversion system according to item 9, wherein a first regenerated flue gas outlet is provided on the regenerator partition.
11. A catalytic conversion system, wherein the catalytic conversion system comprises a first upflow bed reactor, a second upflow bed reactor, a disengager and a regenerator; the first upflow bed reactor has a first catalyst inlet and a first oil catalyst mixture outlet; the second upflow bed reactor has a second catalyst inlet and a second oil catalyst mixture outlet; the first oil catalyst mixture outlet is connected to the lower portion of the second upflow bed reactor;
   the disengager is provided with a settling zone partition and a catalyst separator, wherein the settling zone partition separates the settling zone into a first settling zone and a second settling zone; the catalyst separator has a material inlet connected to the second oil catalyst mixture outlet, a first material outlet opened in the first settling zone, and a second material outlet opened in the second settling zone;
   a regenerator partition is provided in the regenerator, and the regenerator partition separates the regenerator into a first regeneration zone and a second regeneration zone in parallel; a first spent catalyst is transported and connected between the first settling zone and the first regeneration zone; a second spent catalyst is transported and connected between the second settling zone and the second regeneration zone; a first regenerated catalyst is transported and connected between the first regeneration zone and the first upward bed reactor; and a second regenerated catalyst is transported and connected between the second regeneration zone and the second upward bed reactor.
12. A catalytic conversion system according to item 11, wherein the first upflow bed reactor has an upward first catalyst; the second upflow bed reactor has an upward second catalyst and an upward first catalyst; the particle size and density of the first catalyst are greater than the particle size and density of the second catalyst; the first settling zone is located below the settling zone partition, and the second settling zone is located above the settling zone partition.
13. A catalytic conversion system according to item 11 or 12, wherein a first feedstock oil inlet and a fluidized medium inlet are also provided at the lower portion of the first upflow bed reactor, and a second feedstock oil inlet is also provided at the lower portion of the second upflow bed reactor.
14. A catalytic conversion system according to item 11 or 12, wherein the first upflow bed reactor and the second upflow bed reactor are each independently a riser reactor, a fast bed reactor, or a fluidized bed reactor.
15. A catalytic conversion system according to item 11 or 12, wherein the catalyst separator is fixed on the partition plate of the settling zone, and part or all of the second upflow bed reactor is arranged in the first settling zone or the second settling zone.
16. The catalytic conversion system according to item 11 or 12, wherein the catalyst separator is one of or a combination of multiple of a cyclone-type fast separators, a tri-lobe fast separator, a ballistic fast separator, a U-tube separator, and a wall-cutting fast separator, preferably a cyclone-type fast separator;
   the number of the catalyst separators in each of the disengagers is one or more; and the multiple catalyst separators are connected in series and/or in parallel.
17. The catalytic conversion system according to item 11 or 12, wherein a second stripper is provided at the lower portion of the second settling zone; and a second stripping material inlet is provided at the upper portion of the second settling zone.
18. The catalytic conversion system according to item 17, wherein a first stripper is provided in the first spent catalyst transport connection; and a first stripping material outlet is also provided at the upper portion of the first stripper.
19. A catalytic conversion system according to item 11 or 12, wherein the first regeneration zone and the second regeneration zone are arranged side by side on the left and right.
20. A catalytic conversion system according to item 19, wherein the upper portion of the first regeneration zone and the second regeneration zone are connected; the first regeneration zone and the second regeneration zone are respectively provided with a gas-solid separator; and the upper edge of the regenerator partition is higher than the solid outlet of the gas-solid separator.
21. A catalytic conversion method for producing light olefins, wherein the catalytic conversion method comprises the following steps:
   1) contacting the heavy feedstock oil with a first catalyst in a riser reactor to perform a first catalytic conversion reaction to obtain a first oil catalyst mixture;
   2) introducing the first oil catalyst mixture into a fluidized bed reactor to contact with a second catalyst to perform a second catalytic conversion reaction to obtain a second oil catalyst mixture;
   3) subjecting the second oil catalyst mixture to gas-solid separation and stripping in a disengager to obtain an oil and gas product and a mixed spent catalyst;
   4) introducing the mixed spent catalyst into a catalyst separation device for separation to obtain a first spent catalyst and a second spent catalyst, wherein the catalyst separation device comprises a first stage catalyst separator and a second stage catalyst separator;
   5) performing a first regeneration on the first spent catalyst and returning it to step 1) as the first catalyst to participate in the first catalytic conversion reaction, and performing a second regeneration on the second spent catalyst and returning it to step 2) as the second catalyst to participate in the second catalytic conversion reaction.
22. The catalytic conversion method according to item 21, wherein the particle size and density of the first catalyst are greater than the particle size and density of the second catalyst.
23. The catalytic conversion method according to item 21, wherein the first catalyst contains 60-100% by mass of heavy oil catalyst and 0-40% by mass of light oil catalyst; the second catalyst contains 0-40% by mass of heavy oil catalyst and 60-100% by mass of light oil catalyst;
   preferably, the first catalyst contains 80-100% by mass of heavy oil catalyst and 0-20% by mass of light oil catalyst; the second catalyst contains 0-20% by mass of heavy oil catalyst and 80-100% by mass of light oil catalyst.
24. The catalytic conversion method according to item 23, wherein the heavy oil catalyst comprises a modified or unmodified Y-type molecular sieve, clay, a binder, an optional modified or unmodified βmolecular sieve, an optional modified or unmodified ZSM-5 molecular sieve, and an optional heat carrier ;
   based on the total weight of the heavy oil catalyst, the content of the modified or unmodified Y-type molecular sieve is 10-80%, preferably 30-60%, the content of the clay is 10-80%, preferably 15-60%, the content of the binder is 10-30%, preferably 10-20%, the content of the modified or unmodified β molecular sieve is 0-40 % , preferably 0-20% , the content of the modified or unmodified ZSM-5 molecular sieve is 0-40 %, preferably 0-20 %, the content of the heat carrier is 0-40 %, preferably 0-20 %, and the heat carrier is one or a mixture of multiple selected from SiO₂ , MgO, CaO, BaO and MnO₂ ;
   the particle size of the heavy oil catalyst is in the range of 60-250 µm, preferably 80-200 µm, and the particle density is in the range of 1200-1600 kg/m³, preferably 1300-1500 kg/m³.
25. The catalytic conversion method according to item 23 or 24, wherein the light oil catalyst comprises a modified or unmodified ZSM-5 molecular sieve, clay, a binder, an optional modified or unmodified Y molecular sieve, an optional modified or unmodified βmolecular sieve and an optional heat carrier,
   based on the total weight of the catalyst, the content of the modified or unmodified ZSM-5 molecular sieve is 10-60%, preferably 20-50%, the content of the clay is 10-80%, preferably 20-70%, the content of the binder is 10-30%, preferably 10-20%, the content of the modified or unmodified Y molecular sieve is 0-40 %, preferably 0-20%, the content of the modified or unmodified βmolecular sieve is 0-40 %, preferably 0-20 %, the content of the heat carrier is 0-40 %, preferably 0-20 %, and the heat carrier is one or a mixture of multiple selected from SiO₂, MgO, CaO, BaO and MnO₂;
   the particle size of the light oil catalyst is in the range of 10-100 µm, preferably 30-80 µm, and the particle density is 800-1200 kg/m³, preferably 900-1100 kg/m³.
26. The catalytic conversion method according to item 21, wherein the first regeneration zone and the second regeneration zone are arranged in series, the first regeneration zone adopts incomplete regeneration, and the second regeneration zone adopts complete regeneration, and the incompletely regenerated flue gas generated in the first regeneration zone is introduced into the second regeneration zone to continue the regeneration reaction.
27. The catalytic conversion method according to item 21, wherein the regeneration temperature of the first regeneration zone is 640-700°C, preferably 660-680°C, the catalyst distribution density is 50-400 kg/m³, preferably 100-300 kg/m³, and the main air residence time is 0.5-20s, preferably 2-10s;
   the regeneration temperature of the second regeneration zone is 670-730°C, preferably 690-710°C, the catalyst distribution density is 30-350 kg/m³, preferably 80-250 kg/m³, and the main air residence time is 0.5-15s, preferably 2-10s.
28. The catalytic conversion method according to item 21, wherein the reaction temperature of the riser reactor is 520-620°C, preferably 540-600°C; the catalyst-oil ratio is 2-25, preferably 3-20; the reaction time is 1-15 seconds, preferably 2-10 seconds;
   the reaction temperature of the fluidized bed reactor is 540-640°C, preferably 560-620°C, the catalyst distribution density is 20-300 kg/m³, preferably 100-200 kg/m³, the space velocity is 2-15h⁻¹, preferably 5-10h⁻¹; the oil and gas residence time is 0.2-8 seconds, preferably 1-4 seconds.
29. The catalytic conversion method according to item 21, wherein the heavy feedstock is selected from one of or a mixture of vacuum gas oil, atmospheric residue oil, vacuum residue oil, coker gas oil, deasphalted oil, furfural refining raffinate oil, coal liquefaction oil, asphalt, shale oil, Fischer-Tropsch synthesis distillate oil, animal oil and vegetable oil, crude oil, and biomass oil.
30. A catalytic conversion system, wherein the catalytic conversion system comprises a riser reactor, a fluidized bed reactor, a disengager, a stripper, a catalyst separation device, and a regenerator; the upper end of the riser reactor is connected to the lower end of the fluidized bed reactor;
   the upper end of the fluidized bed reactor is connected to the lower end of the disengager; a gas-solid separation device is also provided in the disengager;
   the lower end of the fluidized bed reactor is also connected to a stripper; the solid material outlet of the gas-solid separation device is arranged in the stripper or on the upper portion of the stripper; the lower portion of the stripper has a mixed spent catalyst outlet;
   the catalyst separation device comprises a first stage catalyst separator and a second stage catalyst separator, wherein the first stage catalyst separator has a material inlet connected to the outlet of the mixed spent catalyst, a first spent catalyst outlet and a gas-solid mixture outlet, and the second stage catalyst separator has a material inlet connected to the gas-solid mixture outlet of the first stage catalyst separator, a second spent catalyst outlet and an oil and gas delivery outlet;
   a regenerator partition is provided in the regenerator, and the regenerator partition separates the regenerator into a first regeneration zone and a second regeneration zone; a first spent catalyst is transported and connected between the first spent catalyst outlet and the first regeneration zone; a second spent catalyst is transported and connected between the second spent catalyst outlet and the second regeneration zone; a first regenerated catalyst is transported and connected between the first regeneration zone and the riser reactor; and a second regenerated catalyst is transported and connected between the second regeneration zone and the fluidized bed reactor.
31. A catalytic conversion method for producing ethylene and propylene,
   wherein the catalytic conversion method comprises the following steps:
   1) contacting the heavy feedstock oil with a first catalyst in a first fast bed reactor to perform a first catalytic conversion reaction to obtain a first oil catalyst mixture;
   2) introducing the first oil catalyst mixture into a second fast bed reactor to contact with a second catalyst to perform a second catalytic conversion reaction to obtain a second oil catalyst mixture;
   3) introducing the second oil catalyst mixture into a catalyst separation device for separation to obtain a first spent catalyst and a third oil catalyst mixture;
   4) performing gas-solid separation on the third oil catalyst mixture to obtain an oil and gas product and a second spent catalyst;
   5) the first spent catalyst is subjected to a first stripping and a first regeneration and then returned to step 1) as the first catalyst to participate in the first catalytic conversion reaction, and the second spent catalyst is subjected to a second stripping and a second regeneration and then returned to step 2) as the second catalyst to participate in the second catalytic conversion reaction.
32. The catalytic conversion method according to item 31, wherein the particle size and density of the first catalyst are greater than the particle size and density of the second catalyst.
33. The catalytic conversion method according to item 31 or 32, wherein the first catalyst contains 60-100% by mass of heavy oil catalyst and 0-40% by mass of light oil catalyst; the second catalyst contains 0-40% by mass of heavy oil catalyst and 60-100% by mass of light oil catalyst;
   preferably, the first catalyst contains 80-100% by mass of heavy oil catalyst and 0-20% by mass of light oil catalyst; the second catalyst contains 0-20% by mass of heavy oil catalyst and 80-100% by mass of light oil catalyst.
34. The catalytic conversion method according to item 33, wherein the heavy oil catalyst comprises a modified or unmodified Y-type molecular sieve, clay, a binder, an optional modified or unmodified βmolecular sieve, an optional modified or unmodified ZSM-5 molecular sieve and an optional heat carrier;
   based on the total weight of the heavy oil catalyst, the content of the modified or unmodified Y-type molecular sieve is 10-80%, preferably 30-60%, the content of the clay is 10-80%, preferably 15-60%, the content of the binder is 10-30%, preferably 10-20%, the content of the modified or unmodified β molecular sieve is 0-40 %, preferably 0-20%, the content of the modified or unmodified ZSM-5 molecular sieve is 0-40 %, preferably 0-20 %, the content of the heat carrier is 0-40 %, preferably 0-20 %, and the heat carrier is one or a mixture of multiple selected from SiO₂ , MgO, CaO, BaO and MnO₂;
   the particle size of the heavy oil catalyst is in the range of 60-250 µm, preferably 80-200 µm, and the particle density is in the range of 1200-1600 kg/m³, preferably 1300-1500 kg/m³,
   the particle size of the heavy oil catalyst is in the range of 60-250 µm, preferably 80-200 µm, and the particle density is in the range of 1200-1600 kg/m³, preferably 1300-1500 kg/m³.
35. The catalytic conversion method according to claim 33 or 34, wherein the light oil catalyst comprises a modified or unmodified ZSM-5 molecular sieve, clay, a binder, an optional modified or unmodified Y molecular sieve, an optional modified or unmodified βmolecular sieve and an optional heat carrier,
   based on the total weight of the catalyst, the content of the modified or unmodified ZSM-5 molecular sieve is 10-60%, preferably 20-50%, the content of the clay is 10-80%, preferably 20-70%, the content of the binder is 10-30%, preferably 10-20%, the content of the modified or unmodified Y molecular sieve is 0-40 %, preferably 0-20%, the content of the modified or unmodified βmolecular sieve is 0-40%, preferably 0-20%, the content of the heat carrier is 0-40%, preferably 0-20%, and the heat carrier is one or a mixture of multiple selected from SiO₂, MgO, CaO, BaO and MnO₂ ;
   the particle size of the light oil catalyst is in the range of 10-100 µm, preferably 30-80 µm, and the particle density is 800-1200 kg/m³, preferably 900-1100 kg/m³.
36. The catalytic conversion method according to item 31, wherein the first regeneration and the second regeneration are carried out in the first regeneration zone and the second regeneration zone of the regenerator respectively; the first regeneration zone and the second regeneration zone are arranged in series, the first regeneration zone adopts incomplete regeneration, and the second regeneration zone adopts complete regeneration, and the incompletely regenerated flue gas generated in the first regeneration zone is introduced into the second regeneration zone to continue the regeneration reaction.
37. The catalytic conversion method according to item 31, wherein the regeneration temperature of the first regeneration zone is 640-700°C, preferably 660-680°C, the catalyst distribution density is 50-400 kg/m³, preferably 100-300 kg/m³, and the main air residence time is 0.5-20s, preferably 2-10s;
   the regeneration temperature of the second regeneration zone is 670-730°C, preferably 690-710°C, the catalyst distribution density is 30-350 kg/m³, preferably 80-250 kg/m³, and the main air residence time is 0.5-15s, preferably 2-10s.
38. The catalytic conversion method according to item 31, wherein the reaction temperature of the first fast bed reactor is 520-620°C, preferably 540-600°C; the catalyst-oil ratio is 2-25, preferably 3-20; the reaction time is 1-15 seconds, preferably 2-10 seconds;
   the reaction temperature of the second fast bed reactor is 540-640° C, preferably 560-620° C, the catalyst-oil ratio is 3-30, preferably 5-25; and the reaction time is 1-10 seconds, preferably 2-8 seconds.
39. The catalytic conversion method according to item 31, wherein the heavy raw material is one or a mixture of multiple selected from vacuum gas oil, atmospheric residue oil, vacuum residue oil, coker gas oil, deasphalted oil, furfural refining raffinate oil, coal liquefaction oil, asphalt, shale oil, Fischer-Tropsch synthesis distillate oil, animal oil and vegetable oil, crude oil, and biomass oil.
40. A catalytic conversion system for producing ethylene and propylene, wherein the catalytic conversion system comprises a first fast bed reactor, a second fast bed reactor, a disengager and a regenerator; the upper end of the first fast bed reactor is connected to the lower end of the second fast bed reactor;
   the disengager is provided with a disengager partition and a catalyst separator, wherein the disengager partition separates the disengager into a first settling zone and a second settling zone; the material inlet of the catalyst separator is connected to the upper end of the second fast bed reactor, and the catalyst separator further has a first material outlet opened in the first settling zone and a second material outlet opened in the second settling zone;
   a first stripper is disposed at the lower portion of the first settling zone, and a second stripper is disposed outside the second settling zone;
   a regenerator partition is provided in the regenerator, and the regenerator partition separates the regenerator into a first regeneration zone and a second regeneration zone; a first spent catalyst is transported and connected between the first stripper and the first regeneration zone; a second spent catalyst is transported and connected between the second stripper and the second regeneration zone; a first regenerated catalyst is transported and connected between the first regeneration zone and the first fast bed reactor; and a second regenerated catalyst is transported and connected between the second regeneration zone and the second fast bed reactor.

### Examples

The present application is further described in detail below through examples, but the present application is not limited to these specific examples.

In the following examples and comparative examples:
The gaseous products were analyzed using an Agilent HP5890A refinery gas analyzer, and the liquid products were analyzed by simulated distillation using an Agilent HP6890 gas chromatograph. The cut points for gasoline and diesel were 221°C and 343°C, respectively.

The conversion rate was calculated as: Conversion rate = (weight of dry gas + weight of liquefied gas + weight of gasoline + weight of coke) / weight of fresh feedstock oil feed × 100%;

The yield was calculated as: Ethylene yield = Weight of ethylene product separated from reaction product /weight of fresh feedstock oil feed × 100%; Propylene yield = Weight of propylene product separated from reaction products /weight of fresh feedstock oil feed × 100%.

Unless otherwise specified, the raw materials and reagents used in the following examples and comparative examples are all commercially available products, and the purity of the reagents used is chemically pure.

### Example I series

Three catalysts were used in the I series of examples and comparative examples, namely, catalytic cracking catalyst GOR-II, catalytic cracking catalyst RAG-6, and catalytic cracking catalyst RBC, all of which are commercial catalysts produced by the Qilu Branch of Sinopec Catalyst Company. The specific properties of the three catalysts are shown in Table 1, wherein GOR-II as a heavy oil catalyst was a catalyst containing 40% by weight of Y molecular sieve, RBC was a catalyst containing 33% by weight of Y molecular sieve, 5% by weight of βmolecular sieve and 5% of heat carrier (a mixture of BaO and CaO), and RAG-6 as a light oil catalyst was a catalyst containing 35% by weight of ZSM-5 molecular sieve. Before the test, the catalyst was aged for 17 hours at 800°C and 100% steam.

**Table 1. Composition and properties of catalysts**

| Catalyst | GOR-II | RAG-6 | RBC |
|---|---|---|---|
| Chemical components, %(w) | | | |
| Al₂O₃ | 57.5 | 51.2 | 51.1 |
| SiO₂ | 36.1 | 43.1 | 39.3 |
| BET Full Analysis | | | |
| BET total area/ (m² ·g ⁻¹) | 181 | 197 | 112 |
| Micropore area/ (m² ·g ⁻¹) | 104 | 98 | 66 |
| Total pore volume/ (cm³ ·g⁻¹) | 0.224 | 0.150 | 0.300 |
| Micropore volume/ (cm³ ·g⁻¹) | 0.034 | 0.045 | 0.120 |
| Particle density/ (kg/m³ ) | 1352 | 965 | 1362 |
| Particle size distribution, %(w) | | | |
| 0-20 µm | 0.1 | 0.5 | 0.6 |
| 0-40 µm | 5.1 | 32.6 | 8.1 |
| 0-80 µm | 20.3 | 87.3 | 18.7 |
| 0-105 µm | 50.6 | 98.5 | 47.3 |
| > 105 µm | 49.4 | 1.5 | 52.7 |

The feedstock oils used in the examples and comparative examples of the I series were gas oil and light gasoline. Their specific properties are shown in Tables 2 and 3.

**Table 2. Composition and properties of gas oil**

| Project | Gas oil |
|---|---|
| Density (20°C)/(kg/m³ ) | 856.5 |
| Residual carbon mass fraction/% | 0.12 |
| Element mass composition/% | |
| C | 86.12 |
| H | 13.47 |
| S | 0.85 |
| N | 0.41 |
| Mass group composition/% | |
| Saturates | 83.4 |
| Aromatics | 14.7 |
| Resins | 1.9 |
| Asphaltenes | <0.1 |
| Metal mass composition/(mg/kg) | |
| Fe | 1.9 |
| Ni | 8.0 |
| V | 9.5 |
| Na | 3.1 |
| Ca | 1.8 |
| Distillation range/°C | |
| Initial Boiling point | 284 |
| 10% | 342 |
| 30% | 390 |
| 50% | 420 |
| 70% | 449 |
| 90% | 497 |
| Final Boiling point | 526 |

**Table 3. Composition and properties of light gasoline**

| Project | Light gasoline |
|---|---|
| Density (20°C)/(kg/m³ ) | 635.1 |
| Element mass composition/% | |
| Carbon | 84.76 |
| Hydrogen | 15.24 |
| Sulfur/(µg/g) | 46.29 |
| Nitrogen/(µg/g) | 32 |
| Distillation range/°C | |
| Initial boiling point | 12 |
| 10v% | 18 |
| 30v% | 30 |
| 50v% | 35 |
| 70v% | 57 |
| 90v% | 59 |
| Final boiling point | 60 |
| Mass group composition/% | |
| Paraffins | 37.19 |
| Olefins | 62.49 |
| Naphthenes | 0.32 |
| Aromatics | 0 |

### Example 1-1

The reaction device used in this example (as shown in FIG.3) included a riser reaction zone and a dense phase bed reaction zone connected in series. The inner diameter of the riser reaction zone is 16 mm, and the length was 3200 mm, and the inner diameter of the dense phase bed reaction zone was 64 mm, and the height was 500 mm. The catalyst separator was arranged inside the disengager (as shown in FIG. 12), and the catalyst could be separated into two parts with different particle sizes and particle densities. The type of the catalyst separator is shown in FIG. 6. The preheated gas oil and the first catalyst were introduced into the bottom of the riser reaction zone, and the two contacted and reacted in the riser reaction zone. The reacted oil catalyst mixture was introduced into the dense phase bed reaction zone, and contacted and reacted with the second catalyst therein. The reacted oil catalyst mixture entered the catalyst separator. The catalyst with larger particle size and density was separated to the first settling zone, and the catalyst with a smaller particle size and density was separated to the second settling zone together with the reaction oil and gas. Then they were separated by the cyclone separator in the disengager. The catalyst in the first settling zone and the catalyst in the second settling zone were respectively introduced into the first regeneration zone and the second regeneration zone of the regenerator (as shown in Fig. 13) after stripping. The regenerated catalysts were respectively returned to the riser reaction zone and the dense phase bed reaction zone for recycling. The oil and gas was introduced into the fractionation system for separation. The reaction conditions and results are shown in Table 4.

### Comparative Example I-1

The experiment was conducted according to the method of Example 1-1, except that the catalysts used in the riser reaction zone and the dense phase bed reaction zone were mixed catalysts obtained by mixing GOR-II catalyst and RAG-6 catalyst in a mass ratio of 1:1. The mixed catalyst was added to the dense phase bed reaction zone, and the two catalysts were not separated and regenerated independently in the catalytic conversion system. The reaction conditions and results are shown in Table 4.

### Example 1-2

The experiment was conducted according to the method of Example I-1, except that the catalyst separator was arranged outside the disengager (as shown in FIG.9), the regenerator was of the type shown in FIG. 10, and the preheated light gasoline was introduced into the dense phase bed reaction zone. The reaction conditions and results are shown in Table 4.

### Example 1-3

The experiment was conducted according to the method of Example 1-2, except that RBC catalyst was used instead of GOR-II catalyst as the heavy oil catalyst. The reaction conditions and results are shown in Table 4.

### Comparative Example 1-2

The experiment was conducted according to the method of Comparative Example 1-1, except that the preheated light gasoline was also introduced into the dense phase bed reaction zone. The reaction conditions and results are shown in Table 4.

### Comparative Example 1-3

The reaction device used in this comparative example included a riser reactor and a dense phase bed reactor, which were independent of each other. The diameter of the riser reactor was 16mm and the length was 3200mm, and the diameter of the dense phase bed reactor was 64mm and the height was 500mm. The preheated gas oil and the first catalyst (GOR-II catalyst) were introduced into the bottom of the riser reactor, and they contacted and reacted in the riser reactor. The oil mixture after the reaction was introduced into a cyclone separator for separation to obtain a first reaction product and a first spent catalyst, wherein the first spent catalyst was introduced into the first regenerator for regeneration and recycling. The first reaction product was introduced into a dense phase bed reactor, and contacted and reacted with the second catalyst (RAG-6 catalyst). The oil catalyst mixture after the reaction was introduced into a cyclone separator for separation to obtain a second reaction product and a second spent catalyst, wherein the second spent catalyst was introduced into the second regenerator for regeneration and recycling. The oil and gas were introduced into the fractionation system for separation. The reaction conditions and results are shown in Table 4.

**Table 4. Reaction conditions and results of Examples I-1 and I-2 and Comparative Examples I-1 to 1-3**

| Project | Example I-1 | Compara tive Example I-1 | Example I-2 | Example 1-3 | Compara tive Example I-2 | Compara tive Example I-3 |
|---|---|---|---|---|---|---|
| Riser reaction zone | | | | | | |
| First feedstock oil | Daqing gas oil | Daqing gas oil | Daqing gas oil | Daqing gas oil | Daqing gas oil | Daqing gas oil |
| Catalyst | First catalyst | Mixed catalyst | First catalyst | First catalyst | Mixed catalyst | First catalyst |
| Content of GOR-II in the catalyst/weight % | 73 | 50 | 85 | 85 (RBC) | 50 | 100 |
| RAG-6 content in catalyst/weight % | 27 | 50 | 15 | 15 | 50 | 0 |
| Reaction temperature/°C | 590 | 590 | 580 | 580 | 581 | 580 |
| Catalyst-oil ratio | 12 | 12 | 10 | 10 | 10 | 10 |
| Reaction time/s | 4 | 4 | 4 | 4 | 4 | 4 |
| Dense phase bed reaction zone | | | | | | |
| Second feedstock oil | | | Light gasoline | Light gasoline | Light gasoline | |
| Mass ratio of the first feedstock oil to the second feedstock oil | | | 0.08 | 0.08 | 0.08 | |
| Supplementary catalyst | Second catalyst | Mixed catalyst | Second catalyst | Second catalyst | Mixed catalyst | Second catalyst |
| Content of GOR-II in the catalyst/weight % | 25 | 50 | 18 | 18 (RBC) | 50 | 0 |
| RAG-6 content in catalyst/weight % | 75 | 50 | 82 | 82 | 50 | 100 |
| Reaction temperature/°C | 590 | 590 | 602 | 602 | 600 | 600 |
| Catalyst distribution density | 150 | 150 | 180 | 180 | 180 | 180 |
| Space velocity/h -1 | 4 | 4 | 4 | 4 | 4 | 4 |
| Oil and gas residence time/s | 3 | 3 | 3 | 3 | 3 | 3 |
| Product distribution/wei ght% | | | | | | |
| Dry gas | 8.74 | 9.42 | 9.08 | 9.12 | 9.56 | 9.73 |
| Liquefied gas | 43.53 | 41.77 | 44.69 | 42.88 | 39.06 | 40.68 |
| Gasoline | 27.65 | 26.51 | 24.31 | 25.64 | 25.01 | 24.56 |
| Diesel | 10.42 | 10.13 | 10.15 | 10.87 | 13.58 | 12.47 |
| Slurry oil | 3.49 | 5.22 | 4.98 | 4.65 | 4.25 | 5.44 |
| Coke | 6.17 | 6.95 | 6.79 | 6.84 | 8.54 | 7.12 |
| Conversion rate/weight% | 86.09 | 84.65 | 84.87 | 84.48 | 82.17 | 82.09 |
| Ethylene yield/weight% | 6.13 | 4.52 | 7.11 | 8.02 | 5.56 | 5.46 |
| Propylene yield/weight% | 21.89 | 18.79 | 23.04 | 22.51 | 19.98 | 20.18 |

It can be seen from Table 4 that, compared with the comparative example, the catalytic conversion system and method provided in the present application can increase the yield of preparing light olefins such as ethylene and propylene by cracking heavy crude oil.

### Example II-III series

Three catalysts were used in the following II-III series of examples and comparative examples, namely, catalytic cracking catalyst GOR-II, catalytic cracking catalyst RAG-6 and catalytic cracking catalyst DMMC-2, all of which were commercial catalysts produced by the Qilu Branch of Sinopec Catalyst Company. The specific properties of catalysts GOR-II and RAG-6 are shown in Table 1 above, and the specific properties of catalyst DMMC-2 are shown in Table 5, wherein DMMC-2 was a catalyst containing 15% by weight of Y molecular sieve and 15% by weight of ZSM-5 molecular sieve. Before the test, the catalyst was aged for 17 hours at 800°C and 100% steam.

The feedstock oil used in the examples and comparative examples of series II-III was gas oil, and its specific properties are shown in Table 2 above.

**Table 5. Composition and properties of catalysts**

| Catalyst | DMMC-2 |
|---|---|
| Chemical composition, %(w) | |
| Al₂O₃ | 48.1 |
| SiO₂ | 46 |
| BET Full Analysis | |
| BET total area/(m²·g⁻¹ ) | 102.491 |
| Micropore area/(m²·g⁻¹ ) | 49.601 |
| Total pore volume/ (cm³·g⁻¹) | 0.1057 |
| Micropore volume/(cm³·g⁻¹ ) | 0.0259 |
| Particle density/(kg·m⁻³ ) | 987 |
| Particle size distribution, %(w) | |
| 0-20 µm | 0.1 |
| 0-40 µm | 17 |
| 0-80 µm | 70.9 |
| 0-105 µm | 87.8 |
| > 105 µm | 12.2 |

### Examples II-1 to II-2

The test was carried out on the device shown in Fig. 14, which included a riser reaction zone and a dense phase bed reaction zone connected in series. The inner diameter of the riser reaction zone was 16mm and the length was 3200mm, and the inner diameter of the dense phase bed reaction zone was 64mm and the height was 500mm. The preheated feedstock oil and the first catalyst rich in GOR-II catalyst were introduced into the bottom of the riser reaction zone, and they contacted and reacted in the riser reaction zone. The oil catalyst mixture after the reaction was introduced into the dense phase bed reaction zone, and contacted and continued to react with the second catalyst, which is rich in RAG-6 catalyst. The oil catalyst mixture after the reaction was separated by a cyclone separator in the disengager. The catalyst entered the stripping section for stripping and was introduced into a two-stage catalyst separator. The type of each stage of the catalyst separator is shown in Fig. 7, which was separated into a first spent catalyst rich in GOR-II catalyst and a second spent catalyst rich in RAG-6 catalyst. The two catalysts to be regenerated were introduced into the first regeneration zone and the second regeneration zone, respectively. The regenerated catalysts were returned to the riser reaction zone and the dense phase bed reaction zone for recycling, and the oil and gas were introduced into the fractionation system for separation. The reaction conditions and results are shown in Table 6.

### Comparative Example II-1

The test device used in this comparative example included a riser reaction zone and a dense phase bed reaction zone connected in series. The inner diameter of the riser reaction zone was 16 mm, and the length was 3200 mm, and the inner diameter of the dense phase bed reaction zone was 64 mm and the height was 500 mm. The preheated feedstock oil and DMMC-2 catalyst were introduced into the bottom of the riser reaction zone. They contacted and reacted in the riser reaction zone. The oil catalyst mixture after the reaction was introduced into the dense phase bed reaction zone to continue the reaction. The oil catalyst mixture after the reaction was separated by a cyclone separator in the disengager. The catalyst entered the stripping section for stripping and then entered the regenerator for regeneration. The regenerated catalyst returned to the riser reaction zone for recycling, and the oil and gas were introduced into the fractionation system for separation. The reaction conditions and results are shown in Table 6.

### Comparative Example II-2

The test device used in this comparative example included a riser reaction zone and a dense phase bed reaction zone connected in series. The inner diameter of the riser reaction zone was 16 mm, and the length was 3200 mm, the inner diameter of the dense phase bed reaction zone was 64 mm, and the height was 500 mm. The preheated feedstock oil and a mixed catalyst of GOR-II catalyst and RAG-6 catalyst with a mass ratio of 1:1 were introduced into the bottom of the riser reaction zone. They contacted and reacted in the riser reaction zone. The oil catalyst mixture, after the reaction, was introduced into the dense phase bed reaction zone to continue the reaction. The oil catalyst mixture after the reaction was separated by a cyclone separator in the disengager. The spent catalyst entered the stripping section for stripping and then entered the regenerator for regeneration. The regenerated mixed catalyst returned to the riser reaction zone for recycling. The oil and gas were introduced into the fractionation system for separation. The reaction conditions and results are shown in Table 6.

**Table 6. Reaction conditions and results of Examples II-1 to II-2 and Comparative Examples II-1 to II-2**

| Project | Example II-1 | Example II-2 | Comparative Example II-1 | Comparative Example II-2 |
|---|---|---|---|---|
| Riser reaction zone | | | | |
| First feedstock oil | Daqing gas oil | Daqing gas oil | Daqing gas oil | Daqing gas oil |
| Catalyst | First catalyst | First catalyst | DMMC-2 | First catalyst |
| Content of GOR-II in the catalyst/weight% | 75 | 88 | | 50 |
| Content of RAG-6 in catalyst/weight% | 25 | 12 | | 50 |
| Reaction temperature/°C | 580 | 585 | 582 | 580 |
| Catalyst-oil ratio | 10 | 10 | 10 | 10 |
| Reaction time/s | 4 | 4 | 4 | 4 |
| Dense phase bed reaction zone | | | | |
| Supplementary catalyst | Second catalyst | Second catalyst | None | None |
| Content of GOR-II in the catalyst/weight% | 20 | 11 | | |
| Content of RAG-6 in catalyst/weight% | 80 | 89 | | |
| Reaction temperature/°C | 580 | 604 | 580 | 600 |
| Catalyst distribution density | 150 | 180 | 150 | 180 |
| Space velocity/h ⁻¹ | 4 | 4 | 4 | 4 |
| Oil and gas residence time/s | 3 | 3 | 3 | 3 |
| First regeneration temperature/°C | 680 | 679 | 681 | 680 |
| Second regeneration temperature/°C | 690 | 700 | 699 | 690 |
| Product distribution/weight% | | | | |
| Dry gas | 8.45 | 9.21 | 9.14 | 9.78 |
| Liquefied gas | 41.68 | 42.59 | 37.85 | 40.26 |
| Gasoline | 25.67 | 23.54 | 24.19 | 21.55 |
| Diesel | 14.18 | 13.15 | 15.21 | 14.87 |
| Slurry oil | 3.94 | 3.06 | 5.46 | 4.70 |
| Coke | 6.08 | 8.45 | 8.15 | 8.84 |
| Conversion rate/weight% | 81.88 | 83.79 | 79.33 | 80.43 |
| Ethylene yield/weight% | 5.62 | 6.96 | 5.14 | 4.69 |
| Propylene yield/weight% | 21.47 | 23.08 | 19.38 | 18.22 |

It can be seen from Table 6 that, compared with the comparative example, the catalytic conversion system and method of the present application can increase the yield of light olefins such as ethylene and propylene produced by cracking heavy feedstock oil.

### Examples III -1 to III -2

The test was conducted on the device shown in FIG.15, which included two reaction zones of the type of fast bed reactors connected in series. Each of the reaction zones had a diameter of 40 mm and a length of 1500 mm. The preheated feedstock oil and the first catalyst rich in GOR-II catalyst were introduced into the bottom of the first reaction zone. They contacted and reacted in the first reaction zone. The oil catalyst mixture after the reaction was introduced into the second reaction zone, where it contacted and continued to react with the second catalyst, rich in RAG-6 catalyst. The oil catalyst mixture after the reaction was introduced into the catalyst separator of the type shown in Fig. 7, which was arranged inside the disengager. The oil catalyst mixture was separated into the first spent catalyst rich in GOR-II catalyst and the second spent catalyst rich in RAG-6 catalyst. The two catalysts to be regenerated were introduced into the first regeneration zone and the second regeneration zone, respectively. The regenerated catalysts were returned to the first reaction zone and the second reaction zone for recycling. The oil and gas were introduced into the fractionation system for separation. The reaction conditions and results are shown in Table 7.

### Comparative Example III -1

The device used in this comparative example included two fast bed reactor type reaction zones connected in series. The diameter of the reaction zones was 40 mm and the length was 1500 mm. The preheated feedstock oil and DMMC-2 catalyst were introduced into the bottom of the first reaction zone. They contacted and reacted in the first reaction zone. The oil catalyst mixture after the reaction was introduced into the second reaction zone for further reaction, and the DMMC-2 catalyst was added. The oil catalyst mixture after the reaction was separated by a cyclone separator in the disengager. The spent catalyst entered the regenerator for regeneration after stripping in the stripping section. The regenerated catalyst returned to the first reaction zone for recycling. The oil and gas were introduced into the fractionation system for separation. The reaction conditions and results are shown in Table 7.

### Comparative Example III -2

The device used in this comparative example included two fast bed reactor-type reaction zones connected in series, and the diameter of the reaction zones was 40 mm and the length was 1500 mm. The preheated feedstock oil and the mixed catalyst of GOR-II catalyst and RAG-6 catalyst with a mass ratio of 1:1 were introduced into the bottom of the first reaction zone. They contacted and reacted in the first reaction zone. The oil catalyst mixture after the reaction was introduced into the second reaction zone for further reaction, and the mixed catalyst was added. The oil catalyst mixture after the reaction was separated by a cyclone separator in the disengager. The spent catalyst entered the regenerator for regeneration after stripping in the stripping section. The regenerated mixed catalyst returned to the first reaction zone for recycling. The oil and gas were introduced into the fractionation system for separation. The reaction conditions and results are shown in Table 7.

### Comparative Example III -3

The reaction device used in this comparative example includeed two independent fast bed reactors. The diameter of the reactor was 40mm and the length was 1500mm. The preheated feedstock oil and the first catalyst (GOR-II catalyst) were introduced into the bottom of the first reactor. They contacted and reacted with each other in the first reactor. The oil mixture after the reaction was introduced into a cyclone separator for separation to obtain a first reaction product and a first spent catalyst, wherein the first spent catalyst was introduced into the first regenerator for regeneration and recycling. The first reaction product was introduced into the second reactor, and contacted and reacted with the second catalyst (RAG-6 catalyst). The oil catalyst mixture after the reaction was introduced into a cyclone separator for separation to obtain a second reaction product and a second spent catalyst, wherein the second spent catalyst was introduced into the second regenerator for regeneration and recycling. The oil and gas were introduced into the fractionation system for separation. The reaction conditions and results are shown in Table 7.

**Table 7. Reaction conditions and results of Examples III -1 to III -2 and Comparative Examples III -1 to III -3**

| Project | Exampl e III -1 | Exampl e III -2 | Comparative Example III -1 | Comparativ e Example III -2 | Comparativ e Example III -3 |
|---|---|---|---|---|---|
| First fast bed reactor/zone | | | | | |
| First feedstock oil | Daqing gas oil | Daqing gas oil | Daqing gas oil | Daqing gas oil | Daqing gas oil |
| Catalyst | First catalyst | First catalyst | DMMC-2 | First catalyst | First catalyst |
| Content of GOR-II in the catalyst/weight % | 83 | 77 | | 50 | 100 |
| Content of RAG-6 in catalyst/weight % | 17 | 23 | | 50 | 0 |
| Reaction temperature/°C | 590 | 580 | 582 | 590 | 590 |
| Catalyst-oil ratio | 10 | 10 | 10 | 10 | 10 |
| Reaction time/s | 4 | 4 | 4 | 4 | 4 |
| Second fast bed reactor/zone | | | | | |
| Supplementary catalyst | Second catalyst | Second catalyst | DMMC-2 | Second catalyst | Second catalyst |
| Content of | 18 | 25 | | 50 | 0 |
| GOR-II in the catalyst/weight % | | | | | |
| Content of RAG-6 in catalyst/weight % | 82 | 75 | | 50 | 100 |
| Reaction temperature/°C | 611 | 580 | 580 | 610 | 610 |
| Catalyst to oil ratio | 12 | 10 | 12 | 10 | 10 |
| Reaction time/s | 5 | 5 | 5 | 5 | 5 |
| First regeneration temperature/°C | 680 | 679 | 680 | 680 | 679 |
| Second regeneration temperature/°C | 690 | 690 | 690 | 690 | 690 |
| Product distribution/wei ght% | | | | | |
| Dry gas | 8.91 | 8.16 | 8.45 | 9.37 | 9.43 |
| Liquefied gas | 42.87 | 42.07 | 39.68 | 40.01 | 39.65 |
| Gasoline | 26.41 | 26.12 | 25.61 | 24.59 | 26.24 |
| Diesel | 13.86 | 13.95 | 15.97 | 13.41 | 13.81 |
| Slurry oil | 1.57 | 3.52 | 3.86 | 5.10 | 3.82 |
| Coke | 6.38 | 6.18 | 6.43 | 7.52 | 7.05 |
| Conversion rate/weight% | 84.57 | 82.53 | 80.17 | 81.49 | 82.37 |
| Ethylene yield/weight% | 6.94 | 5.89 | 4.43 | 5.01 | 5.24 |
| Propylene yield/weight% | 22.71 | 21.95 | 18.27 | 19.69 | 20.18 |

It can be seen from Table 7 that, compared with the comparative example, the catalytic conversion system and method provided in the present application can increase the yield of light olefins such as ethylene and propylene produced by cracking heavy feedstock oil.

The preferred embodiments of the present application are described in detail above. However, the present application is not limited to the specific details in the above embodiments. Within the technical concept of the present application, a variety of simple modifications can be made to the technical solution of the present application, and these simple modifications all fall within the protection scope of the present application.

It should also be noted that the various specific technical features described in the above specific embodiments can be combined in any suitable manner without contradiction. In order to avoid unnecessary repetition, this application will not further describe various possible combinations.

In addition, the various implementation modes of the present application may be arbitrarily combined, and as long as they do not violate the concept of the present application, they should also be regarded as the contents disclosed in the present application.

## Claims

1. A fluidized catalytic conversion system, comprising:
a reaction unit, in which the reaction raw materials contact with the catalyst to react to obtain an oil catalyst mixture; the reaction unit comprises a first fluidized bed reactor and a second fluidized bed reactor connected in series, or comprises a composite fluidized bed reactor having a first reaction zone and a second reaction zone connected in series; the first fluidized bed reactor or the first reaction zone of the composite fluidized bed reactor have a first feedstock oil inlet, a first catalyst inlet and a first oil catalyst mixture outlet; the second fluidized bed reactor or the second reaction zone of the composite fluidized bed reactor has an oil catalyst mixture inlet, a second catalyst inlet, a second oil catalyst mixture outlet and an optional second feedstock oil inlet, wherein the first oil catalyst mixture outlet of the first fluidized bed reactor or the first reaction zone of the composite fluidized bed reactor is connected to the oil catalyst mixture inlet of the second fluidized bed reactor or the second reaction zone of the composite fluidized bed reactor;
a catalyst separation unit, in which the oil catalyst mixture from the reaction unit is separated to obtain an oil and gas product and a spent catalyst, the catalyst separation unit comprises a disengager and a catalyst separator arranged inside or outside the disengager, the disengager comprises a gas-solid separator, at least one settling zone and at least one stripping section connected to the bottom of the settling zone, and has an oil catalyst mixture inlet, an oil and gas product outlet and at least one catalyst outlet arranged at the bottom of the stripping section, the catalyst separator has a material inlet, a first material outlet and a second material outlet, wherein the oil catalyst mixture inlet of the disengager is connected to the second oil catalyst mixture outlet of the second fluidized bed reactor or the second reaction zone of the composite fluidized bed reactor; and
a catalyst regeneration unit, in which the spent catalyst from the catalyst separation unit is regenerated and recycled to the reaction unit, the catalyst regeneration unit includes a first catalyst regenerator and a second catalyst regenerator, or includes a composite catalyst regenerator provided with a first regeneration zone and a second regeneration zone, the first catalyst regenerator or the first regeneration zone of the composite catalyst regenerator has a first spent catalyst inlet and a first regenerated catalyst outlet, the second catalyst regenerator or the second regeneration zone of the composite catalyst regenerator has a second spent catalyst inlet and a second regenerated catalyst outlet, wherein the first and second spent catalyst inlets are respectively connected to a catalyst outlet of the disengager, or are respectively connected to the first and second material outlets of the catalyst separator, the first regenerated catalyst outlet is connected to the first catalyst inlet of the first fluidized bed reactor or the first reaction zone of the composite fluidized bed reactor, and the second regenerated catalyst outlet is connected to the second catalyst inlet of the second fluidized bed reactor or the second reaction zone of the composite fluidized bed reactor.

2. The fluidized catalytic conversion system according to claim 1, wherein in the catalyst separation unit:
the catalyst separator is arranged outside the disengager, the catalyst outlet of the disengager is connected to the material inlet of the catalyst separator, the first material outlet of the catalyst separator is connected to the first spent catalyst inlet of the first catalyst regenerator or the first regeneration zone of the composite catalyst regenerator, and the second material outlet is connected to the second spent catalyst inlet of the second catalyst regenerator or the second regeneration zone of the composite catalyst regenerator; or
the catalyst separator is arranged inside the disengager, and the disengager has a first settling zone, a first stripping section connected to the bottom of the first settling zone, a first catalyst outlet arranged at the bottom of the first stripping section, a second settling zone, a second stripping section connected to the bottom of the second settling zone, and a second catalyst outlet arranged at the bottom of the second stripping section, the second oil catalyst mixture outlet of the second fluidized bed reactor or the second reaction zone of the composite fluidized bed reactor is connected to the material inlet of the catalyst separator via the oil catalyst mixture inlet of the disengager, the first material outlet and the second material outlet of the catalyst separator are respectively connected to the first settling zone and the second settling zone of the disengager, the first catalyst outlet of the disengager is connected to the first spent catalyst inlet in the first catalyst regenerator or the first regeneration zone of the composite catalyst regenerator, and the second catalyst outlet of the disengager is connected to the second spent catalyst inlet in the second catalyst regenerator or the second regeneration zone of the composite catalyst regenerator.

3. The fluidized catalytic conversion system according to claim 1 or 2 further comprises a first catalyst injected into the first fluidized bed reactor or the first reaction zone of the composite fluidized bed reactor through the first catalyst inlet, and a second catalyst injected into the second fluidized bed reactor or the second reaction zone of the composite fluidized bed reactor through the second catalyst inlet, wherein the particle size and density of the first catalyst are respectively greater than the particle size and density of the second catalyst.

4. The fluidized catalytic conversion system according to any one of claims 1 to 3, wherein the first fluidized bed reactor and the second fluidized bed reactor, or the first reaction zone and the second reaction zone of the composite fluidized bed reactor, are independently of each other in the form of an upflow bed or a downflow bed, preferably both are in the form of an upflow bed,
preferably, the upflow bed is the type of one or a combination of multiple selected from a bubbling bed, a turbulent bed, a fast bed and a dilute transport bed, preferably a type selected from a riser reactor, a fast bed reactor or a dense phase bed reactor.

5. The fluidized catalytic conversion system according to any one of claims 1 to 3, wherein the first fluidized bed reactor and the second fluidized bed reactor, or the first reaction zone and the second reaction zone of the composite fluidized bed reactor, are independently selected from a riser reactor, a fast bed reactor or a dense phase bed reactor of an upflow bed type,
the first fluidized bed reactor or the first reaction zone of the composite fluidized bed reactor is provided with the first feedstock oil inlet, the first catalyst inlet and the fluidized medium inlet at the lower portion, and the first oil catalyst mixture outlet at the upper portion;
the second fluidized bed reactor or the second reaction zone of the composite fluidized bed reactor is provided with the oil catalyst mixture inlet, the second catalyst inlet, and the optional second feedstock oil inlet at the lower portion, and the second oil catalyst mixture outlet at the upper portion.

6. The fluidized catalytic conversion system according to any one of claims 1-5, wherein the second fluidized bed reactor or the second reaction zone of the composite fluidized bed reactor is of an upflow bed type, and is partially or completely arranged in at least one settling zone of the disengager.

7. The fluidized catalytic conversion system according to any one of claims 1-6, wherein the catalyst separator is one or a combination of multiple selected from cyclone-type fast separator, a tri-lobe fast separator, a ballistic fast separator, a U-tube separator, and a wall-cutting fast separator, preferably a cyclone-type fast separator;
alternatively, the catalyst separator is arranged in the form of a plurality of separators connected in parallel, or in the form of a multi-stage separator connected in series, or a combination thereof, wherein each separator or each stage of the multi-stage separator is independently of each other a cyclone-type fast separator, a tri-lobe fast separator, a ballistic fast separator, a U-tube separator or a wall-cutting fast separator, preferably a cyclone-type fast separator.

8. The fluidized catalytic conversion system according to any one of claims 1-6, wherein the catalyst separator is arranged outside the disengager and is in the form of a two - stage separator connected in series, the inlet of the first-stage separator is connected to the catalyst outlet of the disengager, the first-stage separator is provided with the first material outlet at the lower portion and the light material outlet at the upper portion; the inlet of the second-stage separator is connected to the light material outlet of the first-stage separator, and the second-stage separator is provided with the second material outlet at the lower portion and the residual reaction oil and gas outlet at the upper portion,
optionally, the residual reaction oil and gas outlet of the second-stage separator is connected to the upper portion of the disengager through a pipeline.

9. The fluidized catalytic conversion system according to any one of claims 2-7, wherein the catalyst separator is arranged inside the disengager, and a settling zone partition is provided in the disengager to separate the first settling zone and the second settling zone, the first settling zone is located below the settling zone partition, and the second settling zone is located above the settling zone partition, and the catalyst separator is fixed on the settling zone partition of the disengager so that its first material outlet is located in the first settling zone, and its second material outlet is located in the second settling zone.

10. The fluidized catalytic conversion system according to any one of claims 1 to 9, wherein the composite catalyst regenerator is provided with a regenerator partition separating the first regeneration zone and the second regeneration zone, wherein:
the regenerator partition is arranged vertically, and the first regeneration zone and the second regeneration zone are arranged on the left and right sides of the regenerator partition, optionally, the upper portion of the first regeneration zone and the second regeneration zone are connected, and gas-solid separators are respectively arranged therein, and the upper edge of the regenerator partition is higher than the solid outlet of each of the gas-solid separators; or
the regenerator partition is arranged horizontally, and the first regeneration zone and the second regeneration zone are arranged on the upper and lower sides of the regenerator partition, optionally, the first regeneration zone is arranged below the second regeneration zone, and a first regenerated flue gas outlet is provided on the regenerator partition so that the regenerated flue gas from the first regeneration zone can enter the second regeneration zone.

11. Use of the fluidized catalytic conversion system according to any one of claims 1 to 10 for catalytic conversion of hydrocarbon oil, especially for catalytic conversion of heavy feedstock oil to produce light olefins,
preferably, the heavy feedstock oil is selected from vacuum gas oil, atmospheric residue oil, vacuum residue oil, coker gas oil, deasphalted oil, furfural refining raffinate oil, coal liquefaction oil, asphalt, shale oil, Fischer-Tropsch synthesis distillate oil, animal oil, vegetable oil, crude oil, biomass oil, or a mixture thereof.

12. A method for catalytic conversion of hydrocarbon oil, especially heavy feedstock oil, using the fluidized catalytic conversion system according to any one of claims 1 to 10, comprising the following steps:
1) contacting the hydrocarbon oil feedstock with a first catalyst in a first fluidized bed reactor or a first reaction zone of a composite fluidized bed reactor of a reaction unit of the fluidized catalytic conversion system to perform a first catalytic conversion reaction to obtain a first oil catalyst mixture;
2) contacting the first oil catalyst mixture with a second catalyst in a second fluidized bed reactor or a second reaction zone of a composite fluidized bed reactor of a reaction unit of the fluidized catalytic conversion system to perform a second catalytic conversion reaction to obtain a second oil catalyst mixture;
3) separating the second oil catalyst mixture in a catalyst separation unit of the fluidized catalytic conversion system to obtain an oil and gas product, a first spent spent catalyst, and a second spent spent catalyst;
4) regenerating the first spent spent catalyst in the first catalyst regenerator or the first regeneration zone of the composite catalyst regenerator of the catalyst regeneration unit of the fluidized catalytic conversion system, and returning the obtained first regenerated catalyst to step 1) as the first catalyst; and
5) regenerating the second spent spent catalyst in the second catalyst regenerator or the second regeneration zone of the composite catalyst regenerator of the catalyst regeneration unit of the fluidized catalytic conversion system, and returning the obtained second regenerated catalyst to step 2) as the second catalyst;
preferably, the particle size and density of the first catalyst are respectively greater than the particle size and density of the second catalyst.

13. The method according to claim 12, wherein the first catalyst contains 60-100 mass%, preferably 80-100 mass% of heavy oil catalyst and 0-40 mass%, preferably 0-20 mass% of light oil catalyst, and the second catalyst contains 0-40 mass%, preferably 0-20 mass% of heavy oil catalyst and 60-100 mass%, preferably 80-100 mass% of light oil catalyst, wherein:
based on the total weight of the heavy oil catalyst, the heavy oil catalyst comprises:
10-80%, preferably 30-60%, of modified or unmodified Y-type molecular sieve;
0-40%, preferably 0-20%, of modified or unmodified βmolecular sieve;
0-40%, preferably 0-20%, of modified or unmodified ZSM-5 molecular sieve;
10-80%, preferably 15-60%, of clay;
10-30%, preferably 10-20%, of a binder; and
0-40%, preferably 0-20% of a heat carrier, wherein the heat carrier is selected from SiO₂, MgO, CaO, BaO and MnO₂, or any mixture thereof;
based on the total weight of the light oil catalyst, the light oil catalyst comprises:
10-60%, preferably 20-50%, of modified or unmodified ZSM-5 molecular sieve;
0-40%, preferably 0-20%, of modified or unmodified Y molecular sieve;
0-40%, preferably 0-20%, of modified or unmodified β-structured molecular sieve;
10-80%, preferably 20-70%, of clay;
10-30%, preferably 10-20%, of a binder; and
0-40%, preferably 0-20%, of a heat carrier, wherein the heat carrier is selected from SiO₂, MgO, CaO, BaO and MnO₂, or any mixture thereof,
preferably, the heavy oil catalyst has a particle size range of 60-250 µm, preferably 80-200 µm, and a particle density of 1200-1600 kg/m³, preferably 1300-1500 kg/m³, and the light oil catalyst has a particle size range of 10-100 µm, preferably 30-80 µm, and a particle density of 800-1200 kg/m³, preferably 900-1100 kg/m³.

14. The method according to claim 12 or 13, wherein:
the first fluidized bed reactor or the first reaction zone of the composite fluidized bed reactor is a riser reactor, wherein the reaction conditions include: reaction temperature of 520-620°C, preferably 540-600°C; catalyst-oil ratio of 2-25, preferably 3-20; reaction time of 1-15 seconds, preferably 2-10 seconds; and/or
the second fluidized bed reactor or the second reaction zone of the composite fluidized bed reactor is of the type of dense phase bed reactor, wherein the reaction conditions include: reaction temperature of 540-640°C, preferably 560-620°C, catalyst distribution density of 20-300 kg/m³, preferably 100-200 kg/m³, space velocity of 2-15 h⁻¹, preferably 5-10 h⁻¹; oil and gas residence time of 0.2-8 seconds, preferably 1-4 seconds.

15. The method according to claim 12 or 13, wherein:
the first fluidized bed reactor or the first reaction zone of the composite fluidized bed reactor is a fast bed reactor, wherein the reaction conditions include: reaction temperature of 520-620°C, preferably 540-600°C; catalyst-oil ratio of 2-25, preferably 3-20; reaction time of 1-15 seconds, preferably 2-10 seconds; and/or
the second fluidized bed reactor or the second reaction zone of the composite fluidized bed reactor is of the type of fast bed reactor, wherein the reaction conditions include: reaction temperature of 540-640°C, preferably 560-620 °C, agent-oil ratio of 3-30, preferably 5-25; reaction time of 1-10 seconds, preferably 2-8 seconds.

16. The method according to any one of claims 12 to 15, wherein the first catalyst regenerator or the first regeneration zone of the composite catalyst regenerator adopts incomplete regeneration, and the second catalyst regenerator or the second regeneration zone of the composite catalyst regenerator adopts complete regeneration, the incompletely regenerated flue gas generated in the first catalyst regenerator or the first regeneration zone of the composite catalyst regenerator is introduced into the second catalyst regenerator or the second regeneration zone of the composite catalyst regenerator to continue the regeneration reaction.

17. The method according to any one of claims 12 to 16, wherein:
the regeneration conditions in the first catalyst regenerator or the first regeneration zone of the composite catalyst regenerator include: regeneration temperature of 640-700°C, preferably 660-680°C, catalyst distribution density of 50-400 kg/m³, preferably 100-300 kg/m³, main air residence time of 0.5-20s, preferably 2-10s; and/or
the regeneration conditions in the second catalyst regenerator or the second regeneration zone of the composite catalyst regenerator include: regeneration temperature of 670-730°C, preferably 690-710°C, catalyst distribution density of 30-350 kg/m³, preferably 80-250 kg/m³, main air residence time of 0.5-15s, preferably 2-10s.
